(19) Europäisches Patentamt / European Patent Office / Office européen des brevets

(11) **EP 3 815 743 A1**

(12) **EUROPEAN PATENT APPLICATION**
published in accordance with Art. 153(4) EPC

(43) Date of publication:
**05.05.2021 Bulletin 2021/18**

(51) Int Cl.:
***A61N 5/10*** (2006.01)

(21) Application number: **19822984.1**

(22) Date of filing: **17.06.2019**

(86) International application number:
**PCT/JP2019/023963**

(87) International publication number:
**WO 2019/244854 (26.12.2019 Gazette 2019/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.06.2018 JP 2018115601**
**27.07.2018 JP 2018141896**

(71) Applicant: **NATIONAL INSTITUTES FOR QUANTUM AND RADIOLOGICAL SCIENCE AND TECHNOLOGY**
**Inage-ku**
**Chiba-shi**
**Chiba 263-8555 (JP)**

(72) Inventors:
• **INANIWA, Taku**
**Chiba-shi, Chiba 263-8555 (JP)**
• **NODA, Koji**
**Chiba-shi, Chiba 263-8555 (JP)**
• **IWATA, Yoshiyuki**
**Chiba-shi, Chiba 263-8555 (JP)**

(74) Representative: **Plasseraud IP**
**66, rue de la Chaussée d'Antin**
**75440 Paris Cedex 09 (FR)**

(54) **PARTICLE BEAM IRRADIATION SYSTEM, PARTICLE BEAM IRRADIATION METHOD, IRRADIATIION PLANNING PROGRAM, IRRADIATION PLANNING DEVICE, ELECTROMAGNETIC FIELD GENERATOR, AND IRRADIATION DEVICE**

(57)    Provided is a particle beam therapeutic device, a particle beam therapeutic system, an irradiation planning device, and a particle beam irradiation method. In a particle beam therapeutic system 1, while a magnetic field generation device 9 generates a magnetic field that affects a particle beam in the vicinity of a target of a subject, a particle beam extracted from an ion source 2 is accelerated by an accelerator 4, transported by a beam transport system 5, and applied from an irradiation device 6. In the irradiation of the particle beam, an irradiation planning device 20 creates an irradiation plan of the particle beam in consideration of the influence of the magnetic field, and applies the particle beam based on this irradiation plan.

Fig. 1

**Description**

TECHNICAL FIELD

The present invention relates to a particle beam irradiation system, a particle beam irradiation method, an irradiation planning program, an irradiation planning device, an electromagnetic field generator, and an irradiation device.

BACKGROUND ART

In a particle beam cancer therapeutic device, it is desirable that only a tumor area is damaged when a charged particle beam accelerated by an accelerator applies a certain dose to the tumor area. In order to realize this, it is important to reduce a dose applied to healthy tissues and important organs (healthy tissues/important organs) by a particle beam and secondary particles on a particle beam path and around the path before the particle beam arrives at the tumor area and after the particle beam passes through the tumor area.

When the dose applied to the healthy tissues/important organs other than the tumor area reaches a certain amount, no more dose may be applied to this site, so that the particle beam irradiation cannot be performed. This effect is especially remarkable when the irradiation is performed in only one direction, and in order to reduce this effect, multi-field irradiation or the like is used as an irradiation method.

As for types of the particle beam, a heavy particle beam therapeutic device using a carbon beam or the like is known as a method capable of exerting a sufficiently strong biological effect on an affected site while keeping an influence on tissues around the affected site small.

In a field of this heavy particle beam therapy, therapy using not only the carbon beam but also a helium beam, an oxygen beam, and a neon beam (refer to Non Patent Literatures 1 to 3) and an irradiation method obtained by combining a plurality of nuclides have been proposed (refer to Non Patent Literature 4). The therapeutic methods disclosed in Non Patent Literatures 1 to 4 are irradiation technologies for enhancing a therapeutic effect of the heavy particle beam by optimizing a difference in physical and biological characteristics among respective nuclides.

As another movement, a system that realizes integration of external beam radiation therapy and a magnetic resonance imaging (MRI) device has been proposed, and technologies have been proposed to implement the radiation therapy and image guidance by the MRI without mutual interference (refer to, for example, Patent Literatures 1 to 6).

Patent Literature 1 discloses a magnetic resonance imaging system that determines arrangement of a beam and a magnetic excitation coil assembly in order to avoid the mutual interference and controls them to operate alternately.

Patent Literature 2 discloses a particle beam irradiation device in which a magnetic field generating means includes a plurality of coils configured to allow a particle beam to enter an irradiated region and to generate a uniform magnetic field in the irradiated region with the particle beam, and the magnetic field is perpendicular to an axis (X) of a tubular winding form, in which the magnetic field in a beam direction with small interference is generated.

Patent Literature 3 discloses a particle beam irradiation device configured to irradiate an irradiated region with a particle beam in a predetermined direction, and a relationship between a beam and a magnetic field that enables a particle beam therapy and MRI imaging in parallel.

Patent Literature 4 discloses a system in which a linear accelerator is connected to an MRI device, the system configured to direct particles accelerated in the linear accelerator in a direction along a moving axis of the particles by a magnetic force.

Patent Literature 5 discloses a radiotherapeutic system in which a radiation source and a magnetic resonance diagnostic imaging device simultaneously operate, the system rotating the radiation source in synchronization with the magnetic resonance diagnostic imaging device and adjusting to change timing of operations of the radiation source and MRI, thereby avoiding interference.

The technologies disclosed in Patent Literatures 1 to 5 described above disclose arrangement and control for eliminating the influence of the magnetic field that causes interference on the particle beam irradiation. A method has been proposed on the premise that a high magnetic field of several tens of kilogauss is uniformly applied to the irradiated region for the purpose of MRI image guidance.

CITATION LIST

PATENT LITERATURE

Patent Literature 1: JP 2001-517132 A
Patent Literature 2: JP 2008-543471 A
Patent Literature 3: JP 2008-543472 A
Patent Literature 4: JP 2011-525390 A
Patent Literature 5: JP 2013-146610 A

NON PATENT LITERATURE

Non Patent Literature 1: G.P. Liney, et al., "Technical Note: Experimental results from a prototype high-field inline MRI-linac", Med. Phys., vol. 43 (2016) pp. 5188-5194

Non Patent Literature 2: B. M. Oborn, et al., "Proton beam deflection in MRI fields: Implications for MRI-guided proton therapy", Med. Phys., vol. 42 (2015) pp. 2113-2124

Non Patent Literature 3: T. Tessonnier, et al., "Dosimetric verification in water of a Monte Carlo treatment planning tool for proton, helium, carbon and oxygen ion beams at the Heidelberg Ion Beam Therapy Center", Phys. Med. Biol., Vol. 62 (2017) pp. 6579-6594

Non Patent Literature 4: T. Inaniwa, et al., "Treatment planning of intensity modulated composite particle therapy with dose and linear energy transfer optimization", Phys. Med. Biol., vol. 62 (2017) pp. 5180-5197

Non Patent Literature 5: https://www.gsi.de/en/work/research/biophysics/biophysical_research/physical_modelling_and_treatment_planning.htm)

## SUMMARY OF THE INVENTION

### TECHNICAL PROBLEMS

However, in an irradiation method that combines a heavy particle beam and a plurality of nuclides with a high biological effect, in order to use a carbon beam, and an oxygen beam and a neon beam heavier than the carbon beam, in addition to an ion source from which the carbon beam or a plurality of nuclides may be extracted, a large-scale particle beam facility and an expensive accelerator system are needed to accelerate them to energy that reaches a deep body.

It is desired to control a radiation biological effect so as to irradiate a target area with a high biological effect, whereas minimizing exposure of the surrounding tissue. This is especially required in the field of cancer therapy with high radiation resistance, and is also required for other tumors for higher therapeutic effect and shorter therapeutic period.

The present invention has been achieved in view of such circumstances, and an object thereof is to provide a particle beam irradiation system, a particle beam irradiation method, an irradiation planning program, an irradiation planning device, an electromagnetic field generator, and an irradiation device capable of changing a cell killing effect of a particle beam.

### SOLUTION TO PROBLEMS

The present invention is a particle beam irradiation system including an irradiation device that irradiates an irradiation target with a particle beam, and an irradiation planning device that creates an irradiation plan of the particle beam by the irradiation device, the particle beam irradiation system provided with an electromagnetic field generator that generates a magnetic field or/and an electric field that changes a cell effect that the particle beam applies to the irradiation target.

## ADVANTAGEOUS EFFECTS OF INVENTION

According to the present invention, a cell killing effect of a particle beam may be changed.

## BRIEF DESCRIPTION OF THE DRAWINGS

Fig. 1 is a schematic configuration diagram of a particle beam therapeutic system according to an embodiment of the present invention.
Fig. 2 is a perspective view illustrating a configuration of an electromagnet and an iron shield used in a magnetic field generation device.
Fig. 3A is a vertical sectional view of an electromagnet and an iron core.
Fig. 3B is a cross-sectional view of a hollow conductor forming the electromagnet.
Fig. 4A is a schematic diagram illustrating one conductive wire of a magnetic field generation device.
Fig. 4B is a schematic diagram of an electromagnet in which conductive wires of the magnetic field generation device are stacked.
Fig. 5A is a conceptual diagram of a cell killing effect in a case where no magnetic field is applied.
Fig. 5B is a conceptual diagram of a cell killing effect in a case where a parallel magnetic field is applied.
Fig. 6A is an explanatory view of particle beam irradiation on a highly radiosensitive site of a tumor.
Fig. 6B is an explanatory view of particle beam irradiation to a resistant region of a tumor.
Fig. 7A is a conceptual diagram of a cell killing effect in a case where no magnetic field is applied.
Fig. 7B is a conceptual diagram of a cell killing effect in a case where a parallel magnetic field is applied in the vicinity of a tumor region.
Fig. 8A is a conceptual diagram of a cell killing effect in case where a perpendicular magnetic field is applied in the vicinity of a normal tissue.
Fig. 8B is a conceptual diagram of a cell killing effect in a case where a parallel magnetic field is applied in the vicinity of the tumor and a perpendicular magnetic field is applied in the vicinity of the normal tissue.
Fig. 9 is a cross-sectional view illustrating a state in which a magnetic field is generated by a part of hollow conductors.
Fig. 10 is a conceptual diagram of pencil beam irradiation position (spot).
Fig. 11 is a flowchart of creating an irradiation plan by an irradiation planning device.
Fig. 12 is a flowchart of particle beam irradiation by a particle beam irradiation system.
Fig. 13 is a perspective view of a particle beam irradiation system according to an example 2.

Fig. 14 is a schematic perspective view illustrating an example 3.

Fig. 15 is a schematic configuration diagram of a solenoid electromagnet of the example 3 as seen from above.

Fig. 16 is a schematic partial cross-sectional view of the solenoid electromagnet of the example 3 as seen from the side.

Fig. 17 is a cross-sectional view illustrating a magnetic field calculation example of the solenoid electromagnet of the example 3.

Fig. 18 is a schematic diagram illustrating a schematic configuration of an electromagnet for trunk irradiation of an example 4.

Fig. 19 is a view illustrating an example of calculating magnetic field distribution realized by the electromagnet of the example 4.

Fig. 20 is a conceptual diagram of a parallel magnetic field using two permanent magnets of an example 5.

Fig. 21 is a flowchart of particle beam irradiation by a particle beam irradiation system of an example 6.

Fig. 22A is a view illustrating a dose-surviving fraction curve of cancer cells in a low LET region.

Fig. 22B is a view illustrating a dose-surviving fraction curve of normal cells in the low LET region.

Fig. 23A is a view illustrating a dose-surviving fraction curve of cancer cells in a high LET region.

Fig. 23B is a view illustrating a dose-surviving fraction curve of normal cells in the high LET region.

Fig. 24A is a right side view of a magnetic field generation device of an example 7.

Fig. 24B is a front view of the magnetic field generation device of the example 7.

Fig. 25A is a plan view of the magnetic field generation device of the example 7.

Fig. 25B is a vertical sectional view of the magnetic field generation device of the example 7.

Fig. 25C is a perspective view of a coil of the example 7.

Fig. 26 is a perspective view illustrating a schematic configuration of an electric field generation device of an example 8.

Fig. 27 is a view illustrating a microscopic energy imparting structure around trajectories in water by a proton beam and a carbon beam.

Fig. 28 is a view illustrating a carbon beam cell irradiation experiment in a parallel magnetic field using a solenoid electromagnet.

Fig. 29A is a view illustrating a dose-surviving fraction curve of cancer cells in a result of the carbon beam cell irradiation experiment in the solenoid electromagnet.

Fig. 29B is a view illustrating a dose-surviving fraction curve of normal cells in the result of the carbon beam cell irradiation experiment in the solenoid electromagnet.

Fig. 30 is a view illustrating spiral motion of an electron by the Lorentz force.

DESCRIPTION OF EMBODIMENTS

Hereinafter, embodiments of the present invention are described in detail with reference to the drawings.

[Description of Principle]

First, a basic principle of the present invention is described.

(Background)

A reason why a heavy particle beam represented by a carbon beam is effective in therapy of a tumor in a deep body is that the heavy particle beam intensively emits energy near a stop position (physical characteristic) and has a high cell killing effect in this position (biological characteristic).

A degree of cell killing effect by high-energy charged particles is strongly affected by ionization density in a local region such as a cell and a DNA size. The high-energy charged particles that have just entered the body have a large generation cross-section of high-energy electrons ($\delta$ beam), and they carry out energy far away, so that the ionization density around a trajectory of the charged particles decreases. On the contrary, the charged particles decelerated to several MeV/u near the stop position generate electrons having relatively low energy (hereinafter referred to as secondary electrons), and they emit energy in the vicinity of a generation position, so that the ionization density around the trajectory of charged particles increases. This is one of the reasons why the cell killing effect of the heavy particle beam is further enhanced near the stop position.

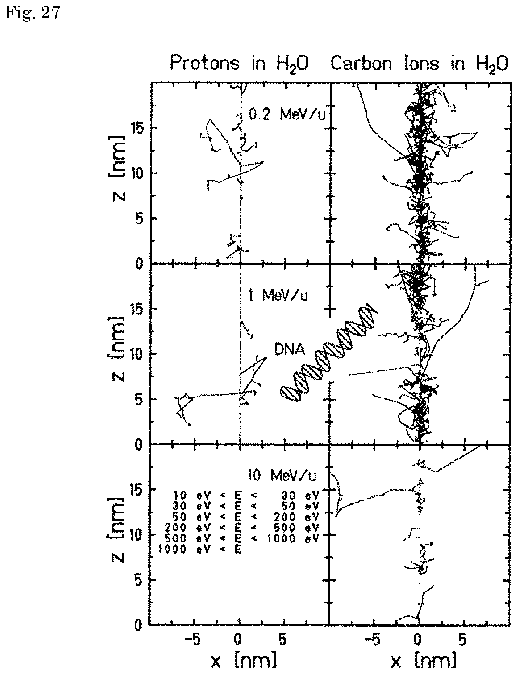

Fig. 27 is a view illustrating a microscopic energy imparting structure around trajectories in water by a proton beam and a carbon beam. Fig. 27 illustrates a result of Monte Carlo simulation of behavior of secondary electrons around the trajectories regarding protons and carbons having energy per nucleon (proportional to a velocity of charged particles) of 10 MeV/u, 1 MeV/u, and 0.2 MeV/u (cited from Non Patent Literature 5).

As illustrated in Fig. 27 described above, the ionization density around the trajectory of the charged particles is high, and the ionization density becomes higher as the energy of the charged particles is smaller, as is particularly remarkable in the carbon beam. In particle beam therapy, a high cell killing effect may be achieved because the charged particles ionize an area around the trajectory with high density and cause an irreparable (fatal) damage to DNA of a cancer cell with high efficiency.

(Experiment)

As described above, with the conventional MRI, arrangement and control for eliminating an influence on both the MRI device and a particle beam irradiation device have been exclusively studied so that a magnetic field thereof does not cause interference with the particle beam irradiation.

In contrast, the present inventors have started a study for specifically confirming the degree of influence of the magnetic field on the cell killing effect of the particle beam therapeutic device, which has not been conventionally studied.

As a result, the present inventors could find a phenomenon that the cell killing effect of the particle beam increases by application of a magnetic field of about several kilogauss parallel to a traveling direction of the particle beam by the following experiment.

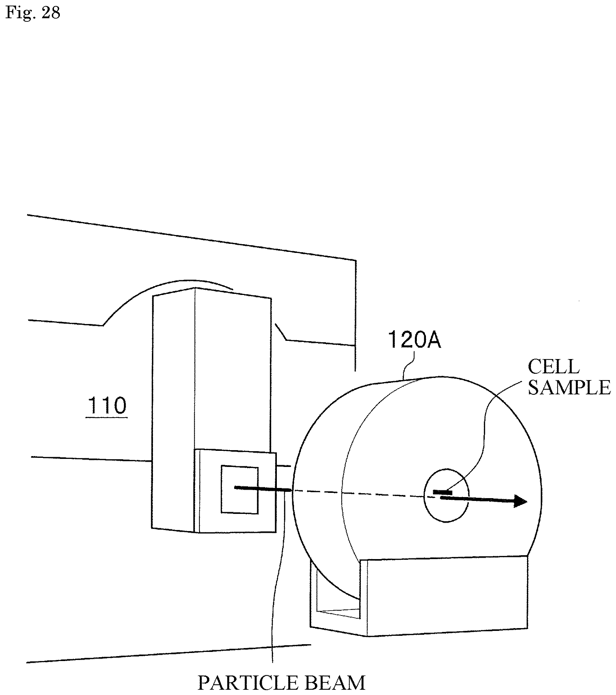

Fig. 28 is a view illustrating a carbon beam cell irradiation experiment in a parallel magnetic field using a solenoid electromagnet. Note that, in this specification, the parallel magnetic field means a magnetic field having a magnetic flux parallel to the traveling direction of the particle beam. Similarly, in this specification, a perpendicular magnetic field means a magnetic field having a magnetic flux perpendicular to the traveling direction of the particle beam.

As illustrated in Fig. 28, the cell irradiation experiment was conducted in which cell samples (cancer cells and normal cells) placed in a solenoid electromagnet 120A were irradiated with a carbon beam (3 MeV/u) by an irradiation device 6. Cell irradiation was performed while changing magnetic field strength in a cell placing position at the center of the solenoid electromagnet 120A to 0, 0.3, and 0.6 T (tesla), and a change in cell killing effect of the carbon beam by the magnetic field was investigated.

In the carbon beam cell irradiation experiment illustrated in Fig. 28, a dose-cell surviving fraction curve was measured under three conditions that (1) no magnetic field is applied by the solenoid electromagnet 120A (0 T), (2) a 0.3 T parallel magnetic field is applied by the solenoid electromagnet 120A, and (3) a 0.6 T parallel magnetic field is applied by the solenoid electromagnet 120A, and the change in cell killing effect of the carbon beam by the magnetic field strength was investigated.

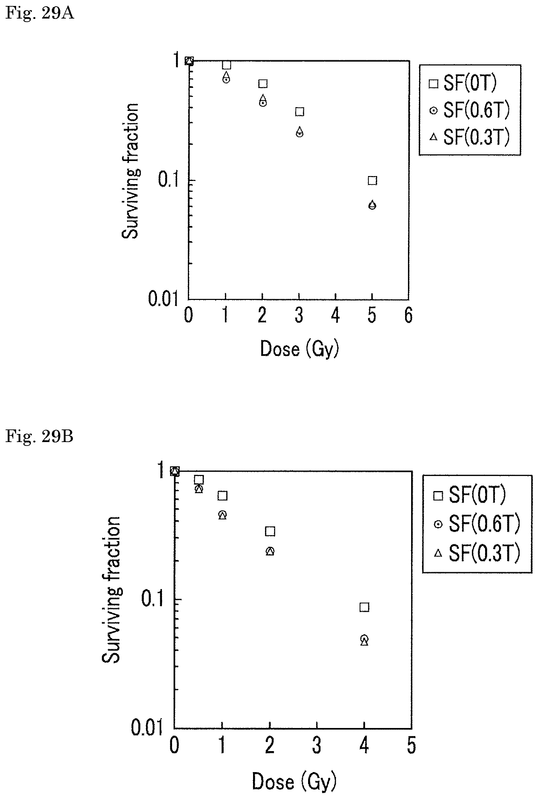

Figs. 29A and 29B are views illustrating results of the carbon beam cell irradiation experiment in the solenoid electromagnet in Fig. 28, in which Fig. 29A illustrates the dose-survival fraction curve of the cancer cells and Fig. 29B illustrates the dose-survival fraction curve of the normal cells. In Figs. 29A and 29B, the cell surviving fraction (rate at which cells survive) is plotted along the ordinate, and the dose of the carbon beam with which the cells are irradiated is plotted along the abscissa. In Figs. 29A and 29B, white squares, double circles, and white triangles indicate the cell surviving fraction with no magnetic field (0 T), with the parallel magnetic field of 0.6 T, and with the parallel magnetic field of 0.3 T, respectively.

As illustrated in Figs. 29A and 29B, regarding both cell types of the cancer cells and normal cells, the cell surviving fraction at the same dose is lower in a case where the parallel magnetic field is applied as compared with a case with no magnetic field applied. This indicates that the cell killing effect of the carbon beam increases by the parallel magnetic field, and more cells may be killed by the application of the magnetic field even when the same dose is applied. It is also found that there is no significant difference in cell surviving fraction between the parallel magnetic fields of 0.3 T and 0.6 T regarding both the cell types of the cancer cells and the normal cells. From this, it is considered that the magnetic field applied to increase the biological effect of about 0.3 T is sufficient.

(Consideration of phenomenon)

The present inventors considered that the effect of increasing the cell killing effect of the carbon beam by the application of the magnetic field parallel to the carbon beam is obtained because the secondary electrons receive the Lorentz force by the magnetic field and a moving range thereof is limited to the vicinity of the trajectory of charged particles, so that the ionization density around the trajectory increases. On the contrary, when the magnetic field perpendicular to the traveling direction of the particle beam is applied, the ionized secondary electrons escape in a direction perpendicular to the trajectory due to the Lorentz force. Therefore, the ionization density around the trajectory decreases.

In detail, many secondary electrons are generated by ionization around the trajectory of the high-energy charged particles. A direction in which the secondary electrons are generated differs depending on the energy of the charged particles and a target type; low-energy electrons (<10 eV) are mainly generated isotropically, and medium to high-energy electrons (>10 eV and <1 keV) are mainly generated in a direction perpendicular to the trajectory of the charged particles.

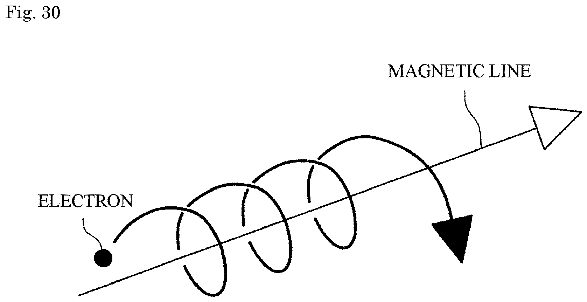

Fig. 30 is a view illustrating spiral motion of an electron by the Lorentz force.

In a case where the magnetic field is applied parallel to the traveling direction of the charged particles, the electron at a velocity of $v\perp$ in the direction perpendicular to the magnetic flux receives the Lorentz force and emits energy while spirally moving around a magnetic line as illustrated in Fig. 30.

A radius of curvature r of the spiral motion of the electron by the Lorentz force is expressed by following equation (1).

[Equation 1]

$$r = \frac{m_e v_\perp}{qB} \quad \cdots (1)$$

me: electron mass

q: elementary charge
B: magnetic field

As is clear from equation (1), the larger the magnetic field B and the smaller the velocity $v\perp$, the smaller the radius of curvature r. That is, the radius of curvature of the spiral motion of the generated electron changes according to magnitude of the applied magnetic field B, and the ionization density around the trajectory of the charged particles changes. Since magnitude of the ionization density around the trajectory significantly affects the degree of cell killing effect of the particle beam, it becomes possible to control the biological effect of the particle beam by appropriately adjusting the applied magnetic field B. In contrast, in a case where the magnetic field B in which the magnetic flux is in the direction perpendicular to the traveling direction of the charged particle is applied, the secondary electrons that receive the Lorentz force travel along the magnetic line while spirally moving and emit energy. In this case, since the secondary electrons move away from the trajectory, it is considered that the ionization density around the trajectory decreases and the cell killing effect of the particle beam decreases.

Since magnitude of the force applied to the electron may be calculated by following equation <Equation 2>, a similar effect may be obtained by controlling (E) corresponding to the electric field as is the case with (B) corresponding to the magnetic field.

<Equation 2>

$$F = qe(E + V * B)$$

* F: force, B: magnetic field, V: velocity at which electron moves,
E: electric field, qe: elementary charge

The present invention is a biological effect variable particle beam irradiation system that maximizes the cell killing effect of the particle beam by appropriately controlling the direction and strength of the magnetic flux or/and the direction and strength of the electric field by utilizing the above-described principle found by the present inventors. Examples of the present invention are described below.

[Example 1]

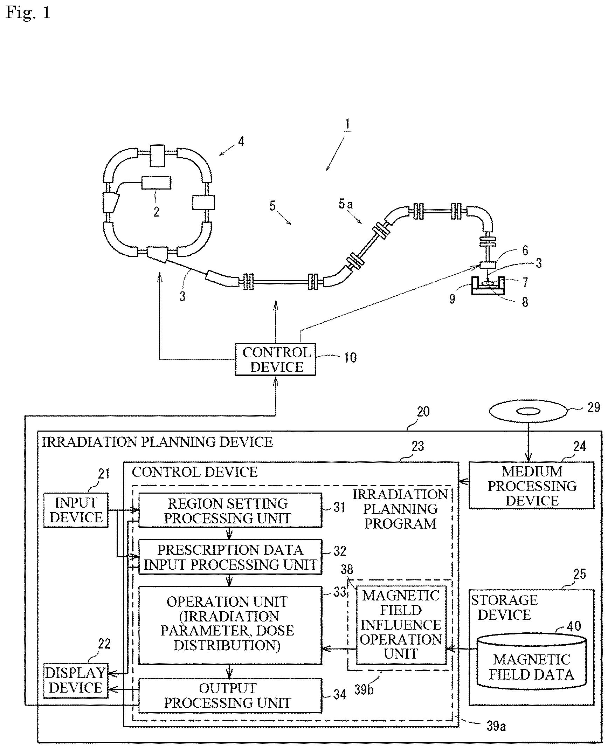

Fig. 1 is an explanatory view illustrating an entire configuration of a particle beam irradiation system 1 (particle beam therapeutic system) according to a first embodiment of the present invention. In this example 1, an example in which a magnetic field generation device 9 is used as an electromagnetic field generator and a cell killing effect of a particle beam is enhanced by a magnetic field is described.

The particle beam irradiation system 1 is provided with an accelerator 4 that accelerates and emits a particle beam 3 (charged particle beam including a charged heavy particle beam) emitted from an injector 2 formed of an ion source and a linear accelerator, a beam transport system 5 that transports the particle beam 3 emitted from the accelerator 4, an irradiation device 6 (scanning irradiation device) that irradiates a target area 8 (for example, a tumor area) to be irradiated of a patient 7 with the particle beam 3 that has passed through the beam transport system 5, the magnetic field generation device 9 that generates the magnetic field in the target area 8 and around the target area 8, a control device 10 (control unit) that controls the particle beam irradiation system 1, and an irradiation planning device 20 as a computer that determines an irradiation parameter of the particle beam irradiation system 1. Note that, a carbon beam is used as the particle beam 3 emitted from the injector 2 in this example, but the present invention is not limited to this and may be applied to the particle beam irradiation system 1 that emits various particle beams.

The accelerator 4 is configured to adjust energy and strength of the particle beam 3.

The beam transport system 5 includes a rotating gantry 5a in the vicinity of the irradiation device 6, and may rotate so as to change an irradiation direction of the particle beam by the irradiation device 6 in a horizontal direction and an arbitrary direction including the horizontal direction. Note that some therapeutic devices are provided with the rotating gantry, while others are provided with only a horizontal or vertical fixed port, and the present invention is applicable to both of them. With the rotating gantry, the irradiation of the particle beam in arbitrary direction in 360 degrees becomes possible.

The irradiation device 6 is provided with a scanning magnet (not illustrated) that deflects the particle beam 3 in an X-Y direction forming a plane perpendicular to a beam traveling direction (Z direction), a dose monitor (not illustrated) that monitors a position of the particle beam 3, and a range shifter (not illustrated) that adjusts a stop position of the particle beam 3 in the Z direction, and scans the particle beam 3 along a scan trajectory with respect to the target area 8. Note that as a method of adjusting a stopping depth of the particle beam, there are a method of adjusting acceleration energy in the accelerator and a method of putting the range shifter in and out on a beam line, or a method of combining both of them, and any method may be used. The range shifter is not required in the method of adjusting the acceleration energy in the accelerator.

The magnetic field generation device 9 is a device that generates the magnetic field in the target area 8 and around the target area 8.

The control device 10 is configured to control the energy and strength of the particle beam 3 from the accelerator 4, position correction of the particle beam 3 in the beam transport system 5, scanning by the scanning magnet (not illustrated) of the irradiation device 6, the beam stop position by the range shifter (not illustrated), the magnetic field generated by the magnetic field generation device 9 and the like.

The irradiation planning device 20 is provided with an input device 21 formed of a keyboard, a mouse and the like, a display device 22 formed of a liquid crystal display, a CRT display or the like, a control device 23 formed of a CPU, a ROM, and a RAM, a medium processing device 24 formed of a disk drive and the like that reads and writes data from and in a storage medium 29 such as a CD-ROM and a DVD-ROM, and a storage device 25 formed of a hard disk and the like.

The control device 23 in the irradiation planning device 20 reads an irradiation planning program 39a and an irradiation plan correcting program 39b stored in the storage device 25, and serves as a region setting processing unit 31, a prescription data input processing unit 32, an operation unit 33, an output processing unit 34, and a magnetic field influence operation unit 38.

The storage device 25 stores the magnetic field generated by an energization state of the magnetic field generation device 9 as magnetic field data 40 for each control unit.

In the irradiation planning device 20 configured in this manner, each functional unit operates as follows according to the irradiation planning program 39a and the irradiation plan correcting program 39b.

The region setting processing unit 31 displays an image of three-dimensional CT value data on the display device 22, and accepts region designation (designation of the target area 8) input by a plan creator using the input device 21.

The prescription data input processing unit 32 displays a prescription input screen on the display device 22 and accept prescription data input by the plan creator using the input device 21. This prescription data is data indicating an irradiation position and an irradiation dose of the particle beam at each coordinate of the three-dimensional CT value data. Note that the prescription data may include particle beam types (for example, carbon nucleus, hydrogen nucleus or the like) and indicate the irradiation position and irradiation dose for each type, and may be made the prescription data using a plurality of types of particle beams.

The operation unit 33 receives the prescription data, magnetic field data, and magnetic field influence data, and creates an irradiation parameter and dose distribution based on them. That is, in order to irradiate the irradiation position of the prescription data with the irradiation dose of the prescription data, the dose of the particle beam (number of particles) to be applied from the particle beam irradiation system 1 is calculated backward using also the magnetic field influence data, and the irradiation parameter of the particle beam to be applied from the particle beam irradiation system 1 is calculated. This irradiation parameter includes particle beam data including the nuclide and dose of the particle beam for each spot to be irradiated and the magnetic field data including magnetic field strength for each spot to be irradiated. Note that there is a case where not only the magnetic field strength but also the direction of the magnetic flux is included, and a case where information indicating a hollow conductor 50 (coil) to be excited is included in the magnetic field data. The operation unit 33 calculates the dose distribution affected by the magnetic field in a case where the irradiation target is irradiated with the particle beam with the calculated irradiation parameter (setting of the particle beam and magnetic field).

The output processing unit 34 outputs and displays the calculated irradiation parameter and dose distribution on the display device 22. The output processing unit 34 transmits the irradiation parameter and dose distribution to a server (not illustrated). At the time of therapy, the irradiation parameter of the therapy is transmitted from the server to the control device 10 that controls the particle beam irradiation system 1. Note that it may also be configured that the output processing unit 34 directly transmits the irradiation parameter to the control device 10 without the intervention of the server.

The magnetic field influence operation unit 38 is configured to calculate the magnetic field generated when the designated magnetic field generation device 9 is energized and an influence of the magnetic field on the particle beam. By calculating the influence of the magnetic field in this manner and transmitting the magnetic field influence data obtained by the calculation to the operation unit 33, it becomes possible to calculate in consideration of the influence of the magnetic field.

With the irradiation planning device 20 configured in this manner, the particle beam irradiation system 1 may apply an effective beam in consideration of the influence of the magnetic field. The beam irradiation may be appropriate irradiation such as spot beam irradiation using a scanning irradiation method in which the target region is irradiated with uniform dose distribution, for example (the dose distribution is the sum of spot beams).

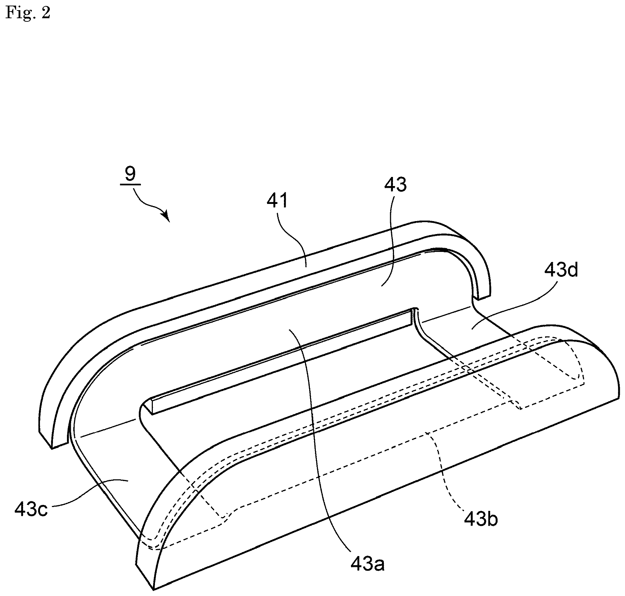

Fig. 2 is a perspective view illustrating a configuration of a coil 43 and an iron shield 41 used in the magnetic field generation device 9. The iron shield 41 is for suppressing a leak magnetic field.

The coil 43 includes longitudinal straight line portions 43a and 43b that are long in a trunk direction of the irradiation target (subject: patient) and arranged in parallel so as to face each other on both sides of the irradiation target, and lateral straight line portions 43c and 43d with which both ends facing each other of the longitudinal straight line portions 43a and 43b are linearly connected in a direction orthogonal to a longitudinal direction of the longitudinal straight line portions 43a and 43b in a position retracted in a direction orthogonal to a facing direction of the longitudinal straight line portions 43a and 43b. The ends of the longitudinal straight line portions 43a and 43b curve at an angle of 90 degrees in an arc shape about the facing direction of the longitudinal straight line portions 43a and 43b, and then curve in an arc shape at an angle of 90 degrees about the longitudinal direction of the longitudinal straight line portions 43a and 43b to be connected to ends of the lateral straight line portions 43c and 43d.

Each surface of the longitudinal straight line portions 43a and 43b other than surfaces facing each other is surrounded by the iron shield 41. The iron shield 41 surrounds, in addition to straight line portions of the longitudinal straight line portions 43a and 43b, a portion in which the ends of the longitudinal straight line portions 43a and 43b curve at the angle of 90 degrees about the facing direction.

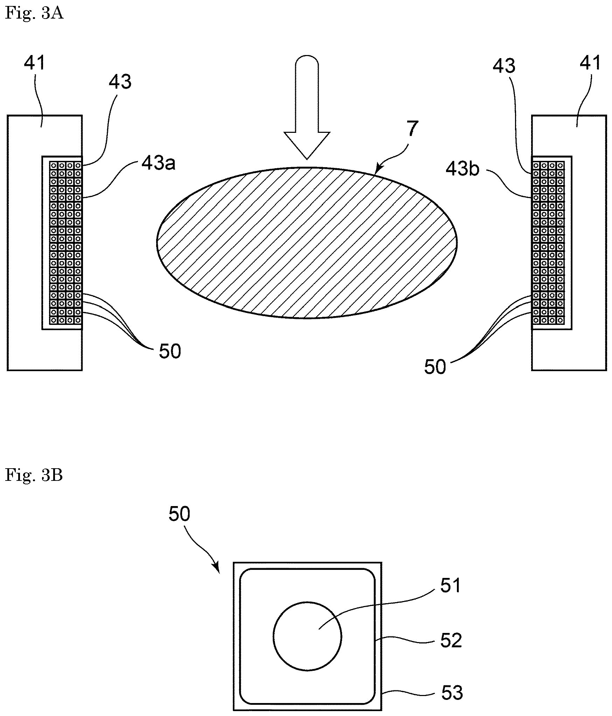

Fig. 3A is a cross-sectional view of the longitudinal straight line portions 43a and 43b and the iron shield 41 taken along a plane a normal of which is in the longitudinal direction of the longitudinal straight line portions 43a and 43b of the coil 43.

In the longitudinal straight line portions 43a and 43b, a plurality of hollow conductors 50 (magnetic field generators) is neatly arranged in a grid pattern in the facing direction of the longitudinal straight line portions 43a and 43b in the cross-section of Fig. 3A and in a direction orthogonal to the direction. In detail, the arrangement number of the hollow conductors 50 in a front-back thickness direction of a body of the patient (direction orthogonal to both the facing direction and longitudinal direction of the longitudinal straight line portions 43a and 43b) is set to the number longer than a thickness length in the front-back thickness direction of a general adult. The arrangement number of the hollow conductors 50 in a lateral width direction of the body of the patient (facing direction of the longitudinal straight line portions 43a and 43b) is set to such the number that the magnetic field by the hollow conductors 50 in the position may sufficiently affect the particle beam. In this example, four columns and twenty columns are arranged in the lateral width direction and the front-back thickness direction of the body of the patient, respectively. In the hollow conductors 50, it is possible to independently apply current for each column and it is possible to form various magnetic fields by applying current of an arbitrary current value to the hollow conductors 50 of an arbitrary column.

The longitudinal straight line portions 43a and 43b are such that three surfaces other than the facing surfaces of the longitudinal straight line portions 43a and 43b are surrounded by the iron shield 41. The iron shield 41 suppresses the leak magnetic field to the outside of the magnetic field generation device.

Fig. 3B illustrates a cross-sectional view of the hollow conductor 50 forming the coil 43.

The hollow conductor 50 is formed of a conductor 52 having a substantially square cross-section with a circular hole 51 at the center, and an insulator 53 covering an outer periphery of the conductor 52. The conductor 52 is made of copper in this example. Cooling water is allowed to flow through the hole 51.

The coil 43 may be used in a case where the tumor in the trunk of the patient fixed on a table in a prone position or supine position is irradiated with the particle beam in the horizontal or vertical direction or arbitrary direction.

Next, the current, magnetic field, and function of the coil 43 configured in this manner are described.

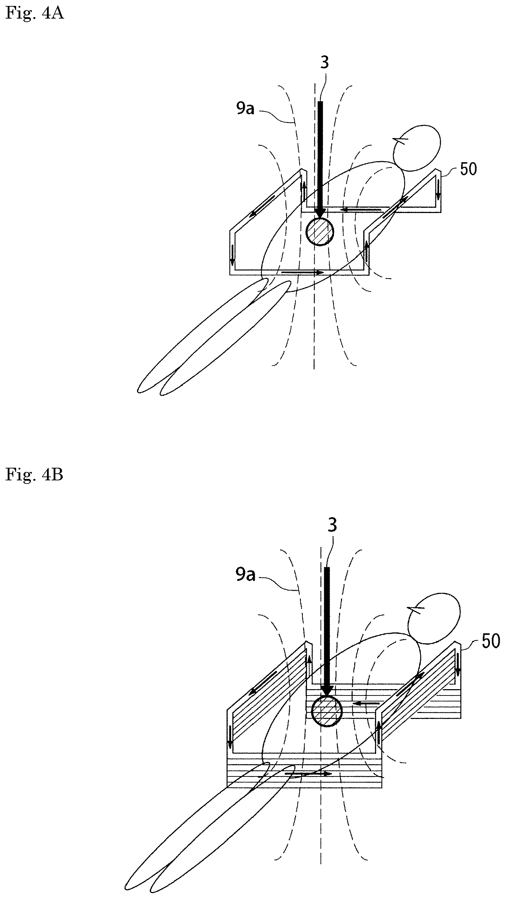

Figs. 4A and 4B are schematic diagrams illustrating a configuration example of the coil 43 for irradiating the trunk, in which Fig. 4A is a perspective view illustrating one conductive wire (hollow conductor 50) out of the coil 43, and Fig. 4B is a perspective view of a state in which the conductive wires (hollow conductors 50) in Fig. 4A are stacked in a beam axis direction (front-back thickness direction of the body of the patient) out of the coil 43. In this example, a magnetic field 9a is indicated by a chain line.

The electromagnet illustrated in Figs. 4A and 4B is attached to a frame of a treatment table, for example, and a mattress and the like is laid on the electromagnet.

In order to explain a current flow in an easy-to-understand manner, Fig. 4A illustrates a schematic electromagnet formed of one conductive wire, and illustrates an image of one conductive wire, the current in the conductive wire, and the magnetic field 9a (magnetic line) generated by the same.

As illustrated in Fig. 4B, the magnetic field generation device 9 (refer to Fig. 1) is provided with the coil 43 obtained by stacking the conductive wires in Fig. 4A in the beam axis direction as a magnetic field generator.

The coil 43 large enough to cover a range (for example, $40 \times 40 \text{ cm}^2$) that may be irradiated by the particle beam irradiation system 1 (refer to Fig. 1) may be configured by passing a return (current in a lateral (right and left) direction) conductive wire under the body. Out of the plurality of conductive wires (hollow conductors 50) forming the coil 43, current is applied only to the conductive wire (hollow conductor 50) in the vicinity of the tumor depth. As a result, as in Figs. 4A and 4B described above, a parallel magnetic field Ba of the magnetic flux in a direction parallel to the particle beam may be realized in the vicinity of the tumor, and a magnetic field in which magnetic flux components in a perpendicular direction increase may be realized in a normal tissue region. There is an effect that it is not necessary to create the solenoid electromagnet that covers the entire body.

In Figs. 4A to 4B, the particle beam is applied in the vertical direction, but the direction in which the particle beam is applied is arbitrary. By rotating the coil 43 (refer to Fig. 2) and rotating the rotating gantry 5a (refer to Fig. 1) according to the direction in which the particle beam is wanted to be applied, the parallel magnetic field Ba may be created for the particle beam in an arbitrary direction.

For example, in a case where the particle beam is wanted to be applied in a lateral direction, it is sufficient to rotate the coil 43 illustrated in Figs. 4A and 4B by 90 degrees or rotate the rotating gantry 5a (refer to Fig. 1) provided on the beam transport system 5 by 90 degrees.

(Magnetic field pattern)

Next, an application pattern of the magnetic field and the cell killing effect are described. In this example, an irradiation plan of the particle beam obtained by appropriately combining the application patterns is created.

«Pattern to apply uniform magnetic field to entire particle beam irradiation region»

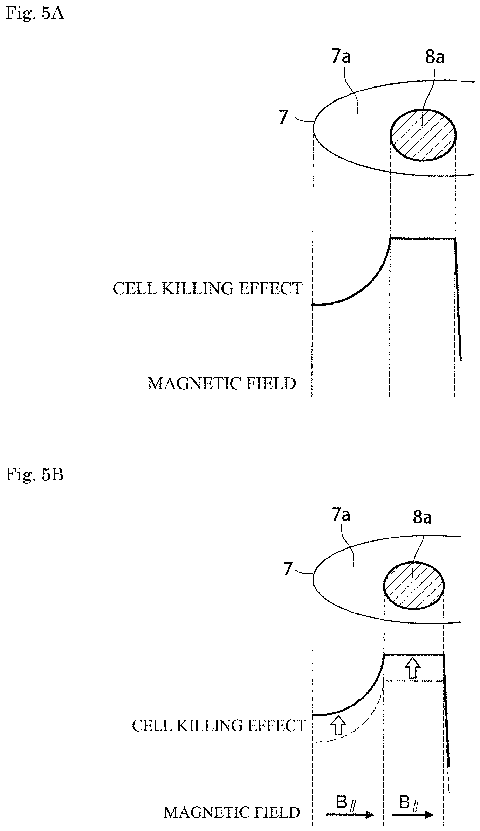

The particle beam irradiation in a case where the uniform parallel magnetic field Ba is applied to an entire particle beam irradiation region (application pattern 1-1) is described in detail with reference to Figs. 5A to 5B.

Note that, in this specification, the term "uniform" regarding the magnetic field means that the direction of the magnetic flux is the same or substantially the same and magnetic flux density is constant or substantially constant in a range in which the magnetic field should be applied.

Figs. 5A and 5B are conceptual diagrams of the cell killing effect (biological effect) in a case where a tumor 8a (refer to hatched portion, the same applies to the following) in the deep body is irradiated with the particle beam from a left side in the drawing, in which Fig. 5A is the diagram of a case where the magnetic field is not applied, and Fig. 5B is the diagram of a case where the uniform parallel magnetic field Ba is applied parallel to the particle beam. In Figs. 5A and 5B, upper parts illustrate the body of the patient, middle parts illustrate the cell killing effect, and lower parts illustrate presence or absence of the parallel magnetic field Ba.

<Application pattern 1-0: No magnetic field is applied>

As illustrated in the middle part in Fig. 5A, the tumor 8a is irradiated with the particle beam (for example, carbon beam) from the left side without the magnetic field applied. In the particle beam irradiation, the irradiation is performed so that the cell killing effect (biological effect) is enhanced at the depth of the tumor 8a. The particle beam intensively emits energy at the tumor 8a (in the vicinity of the stop position) in the deep body, and the maximum cell killing effect (biological effect) may be obtained in the position of the tumor 8a. However, since the particle beam is applied from the left side, there also is the biological effect on a normal tissue 7a near the tumor 8a in the deep body, so that the irradiation in consideration of an allowable range is planned.

<Application pattern 1-1: Entire parallel magnetic field Ba>

On the other hand, as indicated by arrow B// in the lower part in Fig. 5B, in a case where the uniform parallel magnetic field Ba is applied parallel to the particle beam at least while the particle beam enters the patient and arrives at an arrival point by the magnetic field generation device 9 (refer to Fig. 1) of the particle beam irradiation system 1, the cell killing effect of the particle beam is enhanced as indicated by white arrow in the middle part in Fig. 5B. In the example in Fig. 5B, the cell killing effect illustrated in the middle part in Fig. 5A (refer to broken line) is enhanced to the cell killing effect illustrated in the middle part in Fig. 5B (refer to solid line). This increase in cell killing effect has potential to bring about the biological effect substantially similar to that when a helium beam or a carbon beam is used by the parallel magnetic field even in a case where a proton beam is used as the particle beam. That is, it is possible to obtain a therapeutic effect equivalent to that of the helium beam and the like by using a proton beam therapeutic device that is more inexpensive and compact than the heavy particle beam irradiation device.

In this manner, by applying the parallel magnetic field Ba parallel to the beam axis direction of the particle beam, the cell killing effect of the particle beam may be enhanced.

<Usage example in which application patterns 1-0 and 1-1 are combined>

Radiosensitivity in the tumor 8a is not uniform, and a region sensitive to radiation (highly radiosensitive) and a region less sensitive to radiation (radiation resistant) such as a hypoxic region are mixed. In the irradiation method obtained by combining a plurality of nuclides disclosed in Non Patent Literature 4, it is proposed to irradiate the resistant region with oxygen or a neon beam having a higher biological effect.

In the present invention, the cell killing effect of the particle beam may be adjusted by changing the strength of the magnetic field (including the presence or absence of the magnetic field). Furthermore, by changing the strength of the magnetic field to adjust the cell killing effect of the particle beam, it is possible to effectively perform the particle beam therapy in the highly radiosensitive region and the radiation resistant region in the tumor 8a by using one nuclide as in a case where a plurality of nuclides is combined.

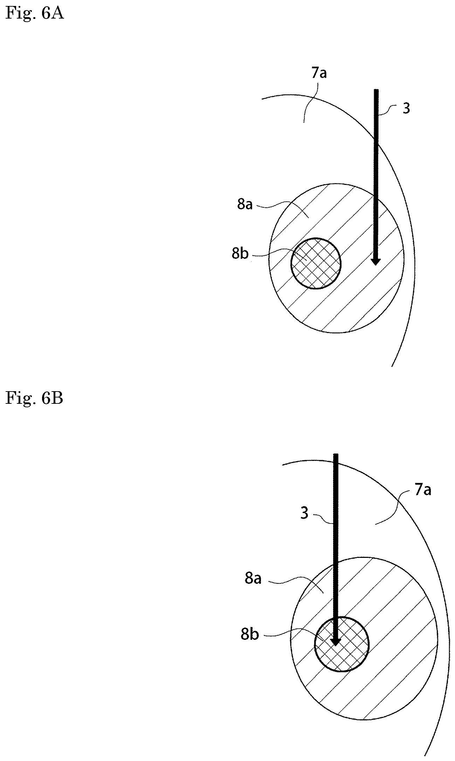

Figs. 6A and 6B are conceptual diagrams illustrating the adjustment of the magnetic field strength of the particle beam irradiation system 1, in which Fig. 6A is the diagram illustrating that no magnetic field is applied when the highly radiosensitive region (refer to hatched portion) of the tumor 8a is irradiated, and Fig. 6B is the diagram illustrating that the uniform parallel magnetic field Ba is applied when the resistant region (refer to grid hatched potion) is irradiated.

As illustrated in Fig. 6A, no magnetic field is applied when the highly radiosensitive portion of the tumor 8a is irradiated. As illustrated in Fig. 6B, the parallel magnetic field Ba is applied only when the radiation resistant region is irradiated. In a case where the highly radiosensitive region and the radiation resistant region are mixed, it is adoptively selected according to the corresponding region between not applying the magnetic field and applying the parallel magnetic field Ba.

By doing so, it is also possible to bring about an effect similar to that of the irradiation method in which a plurality of nuclides is combined. For example, the effect similar to that of the particle beam therapy using a plurality of nuclides to irradiate the highly sensitive region with the carbon beam, and irradiate the resistant region with the oxygen beam and the neon beam may be realized.

«Pattern to apply different magnetic fields for each site of particle beam irradiation region»

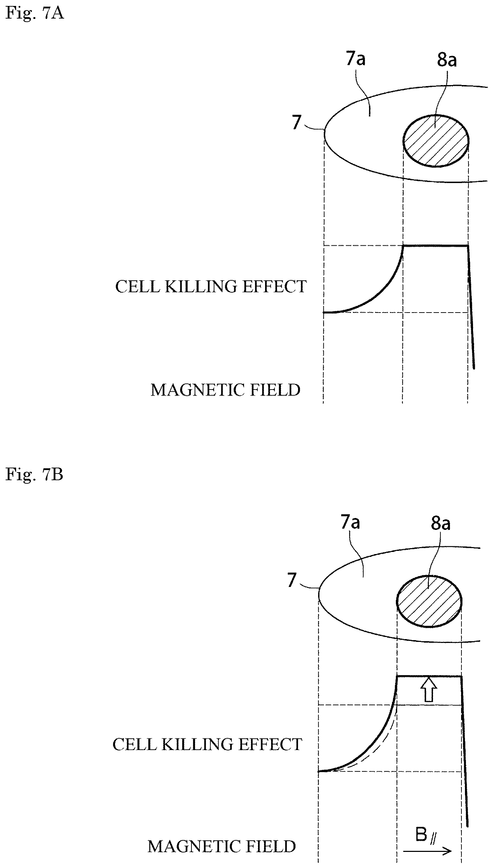

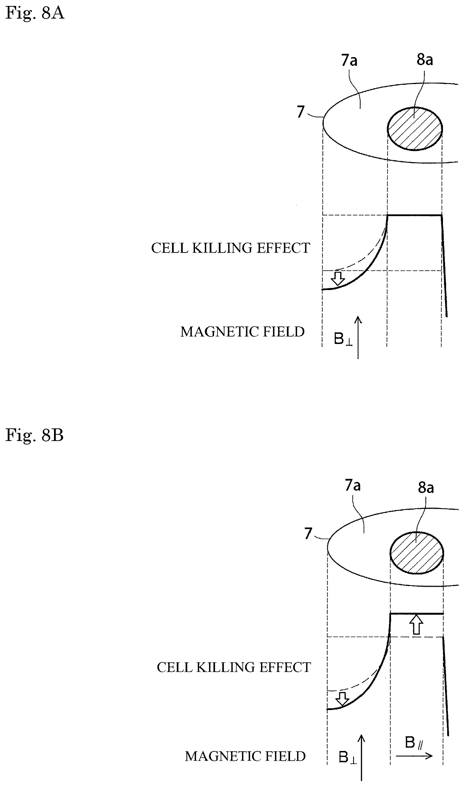

Fig. 7A to Fig. 8B are conceptual diagrams of the cell killing effect (biological effect) in a case where the tumor 8a in the deep body is irradiated with the particle beam from the left side, in which Fig. 7A is the diagram in a case where the magnetic field is not applied, Fig. 7B is the diagram in a case where the parallel magnetic field Ba in the direction parallel to the particle beam traveling direction is applied in the vicinity of the tumor 8a region, Fig. 8A is the diagram of a case where a perpendicular magnetic field Bb is applied so as to be perpendicular to the traveling direction of the particle beam in the vicinity of the normal tissue 7a, and Fig. 8B is the diagram in a case where the parallel magnetic field Ba parallel to the particle beam traveling direction is applied in the vicinity of the tumor 8a and the perpendicular magnetic field Bb which is perpendicular is applied to the vicinity of the normal tissue 7a.

In Figs. 7A to 8B, upper parts illustrate the body of the patient, middle parts illustrate the cell killing effect, and lower parts illustrate the presence or absence of the magnetic field.

<Application pattern 2-0: No magnetic field is applied>

As illustrated in the middle part in Fig. 7A, the tumor 8a is irradiated with the particle beam from the left side without the magnetic field applied. In the particle beam irradiation, the irradiation is performed so that the cell killing effect (biological effect) is enhanced at the depth of the tumor 8a. The normal tissue 7a has no magnetic field. However, since the particle beam is applied from the left side, there also is the biological effect on the normal tissue 7a near the tumor 8a in the deep body to a certain degree.

<Application pattern 2-1: No magnetic field in normal tissue 7a area, parallel magnetic field Ba in tumor 8a area>

As indicated by arrow B// in the lower part in Fig. 7B, the magnetic field generation device 9 of the particle beam irradiation system 1 (refer to Fig. 1) applies the uniform parallel magnetic field Ba parallel to the particle beam in the vicinity of the tumor area. As a result, as indicated by white arrow in the middle part in Fig. 7B, it is possible to enhance the cell killing effect (biological effect) in the tumor region to which the parallel magnetic field Ba is applied by enhancing the cell killing effect of the particle beam.

<Application pattern 2-2: perpendicular magnetic field Bb in normal tissue 7a area and no magnetic field in tumor 8a area>

On the other hand, as indicated by arrow B⊥ in the lower part in Fig. 8A, the magnetic field generation device 9 of the particle beam irradiation system 1 (refer to Fig. 1) applies the perpendicular magnetic field Bb in which the magnetic flux is perpendicular to the traveling direction of the particle beam in the vicinity of the normal tissue 7a (near side). As a result, as indicated by white arrow in the middle part in Fig. 8A, the cell killing effect (biological effect) on the normal tissue 7a is reduced. In the example in Fig. 8A, the cell killing effect on the normal tissue 7a illustrated in the middle part in Fig. 7A (refer to broken line) is reduced to the cell killing effect on the normal tissue 7a illustrated in the middle part in Fig. 8A (refer to solid line). The effect in the tumor region remains unchanged, and the cell killing effect in the normal tissue 7a region to which the perpendicular magnetic field Bb is applied is reduced.

In this manner, by applying the perpendicular magnetic field Bb to the vicinity of the normal tissue 7a, it is possible to reduce the effect on the normal tissue 7a without reducing the cell killing effect on the tumor 8a. It is possible to reduce a risk of damage to the normal tissue 7a.

<Application pattern 2-3: perpendicular magnetic field Bb in normal tissue 7a area and parallel magnetic field Ba in tumor 8a area>

Furthermore, as indicated by arrow B// in the lower part in Fig. 8B and arrow B⊥ in the lower part in Fig. 8B, the magnetic field generation device 9 of the particle beam irradiation system 1 (refer to Fig. 1) applies the uniform parallel magnetic field Ba in which the magnetic fluxes are uniform parallel to the particle beam to the tumor region and applies the uniform perpendicular magnetic field Bb in which the magnetic fluxes are uniform perpendicular to the traveling direction of the particle beam to the normal tissue 7a region. As a result, the cell killing effect on the tumor 8a may be enhanced, and at the same time, the risk of damage to the normal tissue 7a may be reduced. That is, by applying the perpendicular magnetic field Bb to the vicinity of the normal tissue 7a on the near side in the particle beam traveling direction, and applying the uniform parallel magnetic field Ba parallel to the particle beam to the vicinity of the tumor region at the same time, damage to a region outside the target (for example, normal tissue 7a) through which the particle beam passes may be reduced, and the cell killing effect in the vicinity of the target may be further enhanced.

In this manner, by combining the application of various magnetic fields at the time of irradiation of the particle beam, it is possible to create a more desirable irradiation plan.

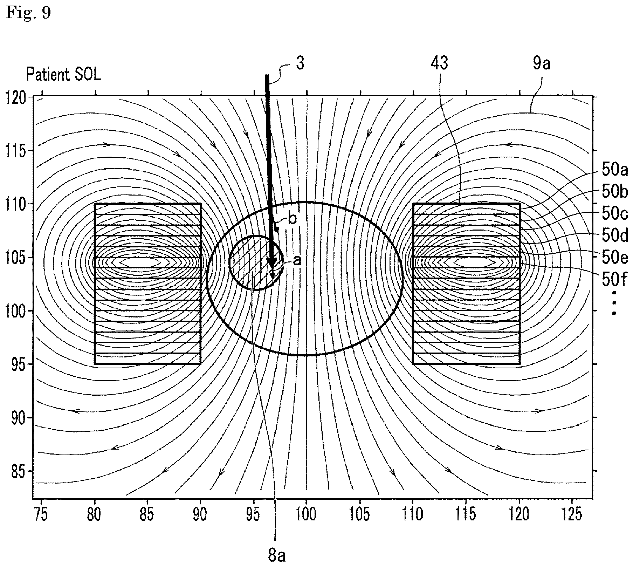

Fig. 9 is a cross-sectional view illustrating a state in which the magnetic field 9a is generated by a part of the hollow conductors 50 regarding the coil 43 in which the hollow conductors 50 are stacked in the irradiation direction of the particle beam 3. Note that, in the illustration in Fig. 9, the iron shield 41 is not illustrated.

In the illustrated example, current is applied to a hollow conductor 50f to generate the magnetic field 9a. The hollow conductor 50f is stacked on a sixth stage from a hollow conductor 50a on an inlet side of the particle beam 3. Therefore, the coil 43 generates the magnetic field 9a by applying current only to the hollow conductor 50f stacked on the sixth stage, and does not apply current to other hollow conductors 50a to 50e and 50g to 50j (not illustrated) and does not generate the magnetic field 9a.

The hollow conductor 50f is configured by arranging a plurality of hollow conductors 50 (refer to Fig. 3A) in one column in the body width direction of the patient (right and left direction in Fig. 9). Therefore, current is applied to all the hollow conductors 50 arranged in one column in a direction orthogonal to the irradiation direction of the particle beam 3 to generate the magnetic field 9a. Note that by changing (increasing or decreasing) a value of the current applied to the hollow conductor 50, the strength of the generated magnetic field 9a may be adjusted (strengthened or weakened).

A depth in the particle beam irradiation direction of the hollow conductor 50 to which the current is applied to generate the magnetic field 9a is preferably set to the same depth as the depth at which the tumor 8a is present (height of the hollow conductor 50 to the side of the tumor 8a), and further, this is preferably set to the same depth as the depth of the beam spot (height of the hollow conductor 50 to the side of the beam spot).

By changing the stage of the hollow conductor 50 to which the current is applied in this manner, it is possible to apply the parallel magnetic field 9a only to the vicinity of the beam spot irradiated with the particle beam 3, and enhance the cell killing effect of this portion as compared with that in other portions.

In detail, the cell killing effect may be enhanced because the parallel magnetic field 9a that is surely parallel is applied to the vicinity of the beam spot, but the magnetic field 9a is not parallel in other portions, so that there is an effect of reducing the cell killing effect as in the perpendicular magnetic field. Therefore, it is possible to weaken the cell killing effect on a portion other than the beam spot at the time of particle beam irradiation, and to perform more desirable particle beam therapy.

By generating the parallel magnetic field at an arbitrary depth in the patient in this manner, a "magnetic field vector in the tumor position" becomes parallel to the beam direction of the particle beam 3 as indicated by arrow a in Fig. 9. Even in the vicinity of the tumor, the parallel magnetic field Ba in which the magnetic line is parallel to the beam traveling direction is strong. As indicated by arrow b in Fig. 9, a "magnetic field vector in the position of the normal tissue 7a" is oblique with respect to the beam direction of the particle beam 3. In the vicinity of the normal tissue 7a, the magnetic line become oblique with respect to the beam traveling direction, so that a parallel component of the magnetic field is weakened, and a perpendicular component is strengthened.

As a result, the magnetic field parallel to the particle beam 3 may be generated in the vicinity of the tumor and the magnetic field in which the components in the perpendicular direction of the magnetic flux increase may be realized in the normal tissue 7a region also in the therapy of the tumor of the trunk.

The strength of the magnetic field applied to the tumor 8a to be irradiated by one column of hollow conductors 50 at the same depth to be excited is about 0.3 tesla in this example. A lower limit of the strength of the magnetic field may be 0.05 tesla or more, preferably 0.1 tesla or more, more preferably 0.2 tesla or more, and 0.3 tesla or more is suitable. An upper limit of the strength of the applied magnetic field may be 5.0 tesla or less, preferably 3.0 tesla or less, more preferably 1.0 tesla or less, and 0.6 tesla or less is suitable.

The magnetic field generation device 9 applies the parallel magnetic field, thereby applying the magnetic field to limit a moving range of the electrons (secondary electrons) ionized by the charged particles being the particle beam 3 to the vicinity of the trajectory of the charged particles in the vicinity of the target. The magnetic field generation device 9 applies the parallel magnetic field, thereby applying the magnetic field to increase the ionization density around the trajectory of the charged particles (particle beam 3) in the vicinity of the target. That is, the magnetic field generation device 9 limits the moving range of the secondary electrons to the vicinity of the trajectory of the charged particles, and increases the ionization density around the trajectory of the charged particles.

Note that by using the coil 43 for irradiating the trunk, a magnetic field shape illustrated in Fig. 9 may be created, so that there is a peculiar effect that it becomes not necessary to create the solenoid electromagnet in a ring shape that covers the entire body.

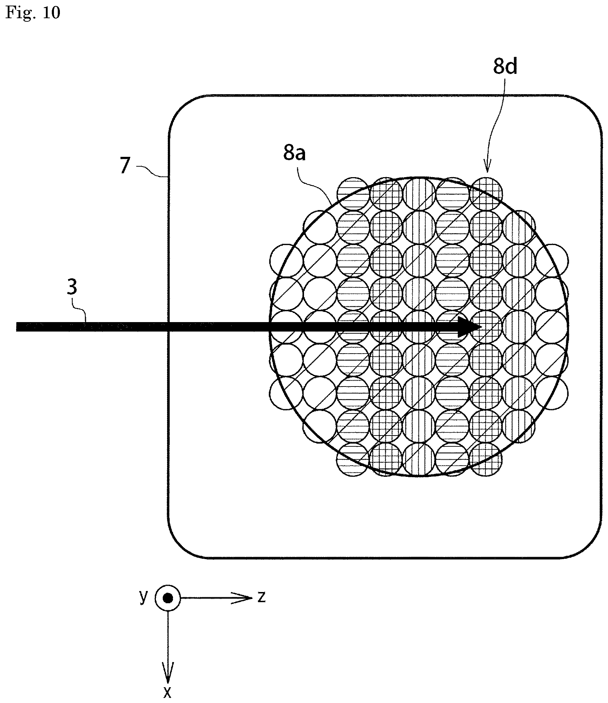

Fig. 10 is a conceptual diagram of a pencil beam irradiation position (spot) as seen by slicing the target and patient in a particle beam scanning irradiation method using the particle beam irradiation system 1. Circles of thin solid lines in Fig. 10 indicate the pencil beam irradiation positions (spots). The illustrated slice illustrates an XZ plane in a three dimensional axis in which the particle beam irradiation direction (depth direction) in a case of the irradiation of the particle beam horizontally is a Z axis, a gravity direction orthogonal to the Z axis is an X axis, and a direction orthogonal to both the Z axis and X axis is a Y axis. As illustrated in the drawing, the spots are arranged vertically and horizontally at equal intervals without gaps in this XZ plane (a plurality of spots is arranged in each slice). The spots arranged in this manner are similarly arranged at equal intervals without gaps in a Y-axis direction, and the spots are neatly arranged three-dimensionally at equal intervals without gaps. The magnetic field and the particle beam may be changed for each spot, and for example, it is possible to apply a different magnetic field according to each type of hatching indicated in the circle to irradiate each spot with the particle beam.

In the scanning irradiation method, a thin particle beam pencil beam accelerated by the accelerator 4 of the particle beam irradiation system 1 (refer to Fig. 1) is three-dimensionally scanned according to the tumor shape, and dose distribution of the pencil beam with which each position is irradiated is overlapped, thereby realizing desired dose distribution in the patient.

In order to apply a necessary and sufficient dose into the target, the irradiation positions (spots) of the pencil beam are spread in the target, and the position is irradiated with the number of particles determined by the irradiation plan. Scanning of the pencil beam in the depth direction (Z direction) is performed by changing the velocity (kinetic energy) of the particles accelerated by the accelerator.

In contrast, the scanning of the slice in the XY plane is performed by exciting an electromagnet referred to as a scanning electromagnet in the irradiation device 6 (refer to Fig. 1) installed several meters upstream from an isocenter that is the irradiation center of the particle beam. In general irradiation, an innermost slice is irradiated first, and after all the spots in the slice are irradiated, the kinetic energy of the particle beam is lowered by the accelerator, and a second innermost slice is irradiated. This is repeated until the spots of all the slices are irradiated.

Irradiation of each field (beam) is managed in spot unit, so that it is possible to switch such that a certain hollow conductor 50 (refer to Fig. 3A) of the coil 43 (refer to Fig. 2) is excited in a case where a certain spot is irradiated.

Next, an irradiation planning workflow of the particle beam scanning irradiation method of this embodiment is described.

<Irradiation planning workflow of this embodiment>

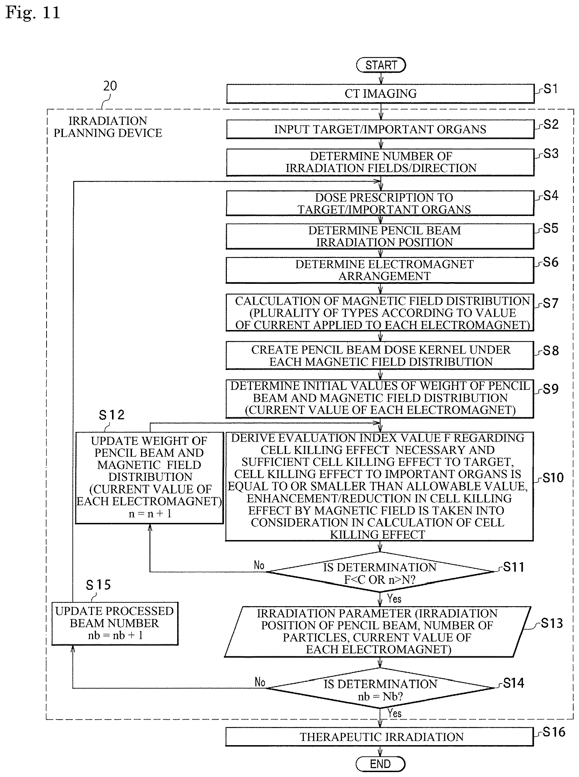

Fig. 11 is a flowchart illustrating a workflow of the irradiation planning device 20 in consideration of "modulation of the particle beam biological effect by the magnetic field" proposed in this embodiment. In Fig. 11, work on the irradiation planning device 20 is enclosed by broken line.

A thin particle beam pencil beam accelerated by the accelerator and transported to a treatment room is applied while three-dimensionally scanning according to the shape of the tumor 8a.

The particle beam scanning irradiation method is the irradiation method of the particle beam therapy that creates desired dose distribution in the patient.

As illustrated in Fig. 11, the irradiation planning device 20 draws an irradiation plan for the particle beam irradiation based on the cell killing effect (modulation of the biological effect) by the magnetic field according to the irradiation planning program 39a and the irradiation plan correcting program 39b. This irradiation plan determines the position (beam spot), the nuclide, and the dose of the particle beam to be applied for realizing the desired dose distribution in the patient in the particle beam scanning irradiation method in which the particle beam irradiation system 1 described with reference to Fig. 1 makes the particle beam accelerated by the accelerator 4 the thin pencil beam by the irradiation device 6 to apply while three-dimensionally scanning according to the tumor shape.

First, the control device 23 (refer to Fig. 1) of the irradiation planning device 20 captures a CT image of an affected site taken by an appropriate CT device (step S1).

The control device 23 visualizes the target and important organs on the captured CT image by the region setting processing unit 31 (step S2).

The control device 23 determines the number of fields of the particle beam irradiation (the number of irradiation directions (Nb)) and the directions thereof for the target by the region setting processing unit 31 (step S3).

The control device 23 accepts an input of a dose prescription regarding the target and important organs by the prescription data input processing unit 32 to determine (step S4). The dose prescription is the dose with which the target should be irradiated and an allowable dose regarding the important organs. That is, a position in the tumor, the dose to be applied at least, and the allowable dose to the important organs other than the tumor are determined as the dose prescription. The dose herein referred to is an index indicating a degree of cell killing effect by the particle beam irradiation.

The control device 23 determines the irradiation position of the particle beam pencil beam that should be applied in order to give the target a necessary and sufficient dose by the prescription data input processing unit 32 (step S5).

The control device 23 determines electromagnet arrangement after the irradiation position of the particle beam pencil beam is determined at step S5 and before creating a pencil beam dose kernel by the operation unit 33 (step S6). In this electromagnet arrangement, for example, the coil 43 for irradiating the trunk in Fig. 2 described above is installed according to the position of the target and the beam irradiation direction. Note that various electromagnets may be used as the electromagnet to be arranged; for example, in a case where a ring-shaped head and neck tumor irradiation solenoid electromagnet is used, the head and neck tumor irradiation solenoid electromagnet is installed.

The control device 23 calculates magnetic field distribution realized in the body of the patient by arrangement of the installed electromagnet by the magnetic field influence operation unit 38 (irradiation plan correcting program 39b) (step S7). However, for example, the magnetic field distribution differs depending on the hollow conductor 50 to be excited and the value of the current applied thereto, so that the magnetic field distribution is calculated for a plurality of combination candidates. This magnetic field distribution is determined by the shape, position, and current value of the hollow conductor 50, and it may also be configured to selectively use the magnetic field distribution calculated in advance as distribution data for each shape, position, and current value. Note that the magnetic field distribution for each current value for each hollow conductor 50 to be excited is preferably calculated or measured in advance to be stored in the storage device 25.

The control device 23 calculates the pencil beam dose kernel under each magnetic field distribution by the operation unit 33 (step S8). This calculation may be performed by an appropriate method such as by calculating by using the magnetic field influence data prepared in advance by experiments and calculation of how much cell killing effect the particle beam of a certain dose exerts with respect to the direction and strength of the magnetic flux. However, since the magnetic field strength applied by the magnetic field generation device 9 is weak (for example, 0.3 T or less), it is predicted that distortion (bending and rotation) of the particle beam due to the magnetic field is small. For this reason, there is a case where it is sufficient that the pencil beam dose kernel is calculated without a magnetic field.

The control device 23 determines initial values of the number of particles (weight) of the pencil beam and the magnetic field distribution (current value of each electromagnet) by the operation unit 33 (step S9).

The control device 23 performs optimal determination of the number of particles of the pencil beam with which each position is irradiated in order to satisfy the designated dose prescription and the value of current applied to each electromagnet during the pencil beam irradiation by successive approximation repetitive calculation by the operation unit 33 (steps S10 to S12).

The control device 23 calculates an evaluation index value F related to the cell killing effect by the operation unit 33 (step S10). As the evaluation index value F, a target cell killing effect value for determining that there is a necessary and sufficient cell killing effect for the target, and an important organ cell killing effect value for determining that the cell killing effect on the important organs is equal to or smaller than the allowable value are calculated in consideration of improvement/reduction of the cell killing effect by the magnetic field.

The control device 23 determines whether the successive approximation repetitive calculation satisfies a predetermined dose prescription (F<C) or exceeds a predetermined fixed number of repetitive calculation (n>N) by the operation unit 33 (step S11).

In a case where the successive approximation repetitive calculation does not satisfy the predetermined dose prescription, or does not exceed the predetermined fixed number of repetitive calculation (step S11: No), the control device 23 increments the number of repetitive calculation n (n = n + 1) for updating the number of particles of the pencil beam and the magnetic field distribution (current value of each electromagnet) by the operation unit 33 and returns to step S10 (step S12).

The control device 23 finishes repetition to shift to a next process by the operation unit 33 in a case where the successive approximation repetitive calculation satisfies the predetermined dose prescription or exceeds the predetermined fixed number of repetitive calculation (step S11: Yes).

The control device 23 makes "the number of particles of the pencil beam with which each position is irradiated and the value of current applied to each electromagnet during the pencil beam irradiation" at that time the irradiation parameter for this field by the operation unit 33 (step S13).

The control device 23 determines whether or not the irradiation plan for a predetermined number of fields (Nb) is completed (nb = Nb) by the operation unit 33 (step S14).

In a case where the irradiation plan for the predetermined number of fields (Nb) is not completed (step S14: No), the control device 23 determines that it is insufficient and updates the processed beam number nb (step S15), increments the number of repetitive calculation n (nb = nb + 1) by the operation unit 33, and repeats the procedure from step S4.

In a case where the irradiation plan of the predetermined number of fields (Nb) is completed (step S14: Yes), the control device 23 proceeds to therapeutic irradiation of the particle beam by the particle beam irradiation system 1 by the operation unit 33 (step S16).

The irradiation planning workflow by the irradiation planning device 20 is finished above, and the irradiation parameter is output to the control device 10 (refer to Fig. 1) of the particle beam irradiation system 1.

After that, the control device 10 (refer to Fig. 1) of the particle beam irradiation system 1 executes the therapeutic irradiation based on the irradiation parameter drawn by the irradiation planning device 20.

[Irradiation workflow: rotating gantry chamber]

Next, the irradiation workflow with the gantry port using the particle beam irradiation system 1 is described. Many proton beam therapeutic facilities are equipped with the rotating gantry, which allows beam irradiation in arbitrary direction in 360 degrees around the patient. The rotating gantry is an irradiation port provided with a rotating mechanism rotatable around the patient. There are only few therapeutic facilities equipped with the rotating gantry as carbon beam therapeutic facilities; this operates only in the NIRS and in the Heidelberg Ion Beam Therapy Center in the world. In many carbon beam facilities, the beam irradiation is available only in a certain direction fixed to the treatment room: horizontal port, vertical port, and horizontal + vertical port.

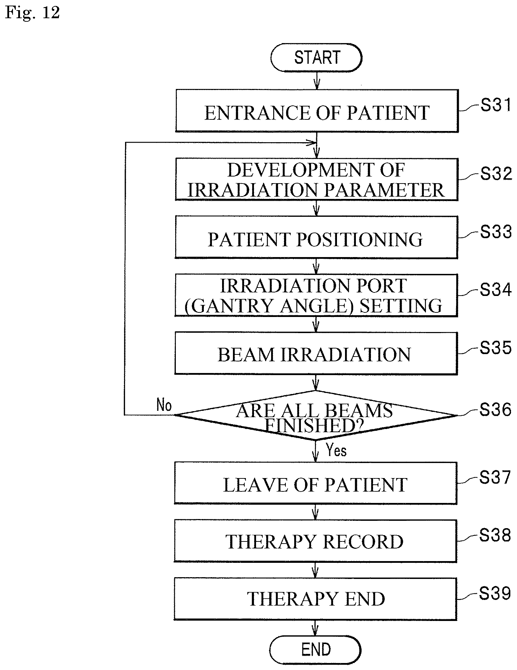

Fig. 12 is a flowchart illustrating an irradiation workflow of irradiation of the particle beam in the rotating gantry chamber using the particle beam irradiation system 1.

The control device 10 of the particle beam irradiation system 1 obtains the irradiation parameter from the server to develop (step S32) after it is input that the patient enters the rotating gantry chamber (step S31). The irradiation parameter obtained at that time is obtained in one field (beam) unit of beam irradiation. Note that the irradiation parameter may be obtained directly from the output processing unit 34 without the intervention of the server.

The control device 10 accepts patient positioning for the beam irradiation (step S33). The control device 10 accepts setting of the irradiation port (gantry angle) and the like (step S34).

After the positioning is approved, the control device 10 starts the beam irradiation (step S35). In this beam irradiation, a prescribed magnetic field 9a is applied to each spot in the three-dimensional position of the tumor 8a, and the prescribed dose of particle beam 3 is applied while the magnetic field 9a is applied. At that time, the magnetic field generation device 9 continuously applies a uniform magnetic field 9a at least from when the irradiation of the particle beam 3 is started to one spot until the irradiation is completed. The application of the magnetic field 9a and the irradiation of the particle beam 3 are executed to all the spots in spot unit according to the irradiation plan (irradiation parameter) planned for the current field by the irradiation planning device 20.

When the irradiation of all the beams is not completed (step S36: No), the control device 10 returns the procedure to step S32 to repeat. After this repetition, for example, in a case of a second field, the irradiation parameter of the second field is developed in the control device 10. In a case where an installation position of the patient does not change from that in the irradiation in the preceding field; for example, the installation position of the patient does not change between the first field and the second field, the control device 10 skips the patient positioning at step S33 (accept that the position is the same as that at the time of the irradiation in the preceding field).

In a case where all the beams are completed (step S36: Yes), the control device 10 allows the patient to leave the chamber (step S37). For example, in a case where the irradiation of all the beams scheduled for the patient on that day is finished, the patient leaves.

The control device 10 stores a therapy record such as an irradiation log in an appropriate storage unit (step S38) and accepts the end of the therapy (step S39).

By the above-described configuration and operation, in the particle beam irradiation system 1 (refer to Fig. 1), the irradiation device 6 and the magnetic field generation device 9 may generate the magnetic field that affects the cell killing effect by the particle beam in the vicinity of the target of the subject and apply the particle beam under an environment of the magnetic field to change the cell killing effect. That is, it is possible to change (differentiate) the cell killing effect by the magnetic field with the irradiation of the same nuclide and the same dose without changing the nuclide of the particle beam to be applied or the irradiation dose. Therefore, a degree of freedom in therapy plan may be increased, and functionality of the particle beam irradiation system 1 may be improved.

It is possible to apply the particle beam while generating a magnetic field in which a magnetic flux is in a direction parallel to a beam axis, thereby enhancing the cell killing effect by the particle beam.

The irradiation device 6 and the magnetic field generation device 9 may irradiate the normal tissue 7a of the subject with the particle beam while generating a magnetic field in which the magnetic flux is in a direction perpendicular to or intersecting with a beam axis of the particle beam, and reduce the cell killing effect of particle beam 3 on the normal tissue 7a.

In this manner, it is possible to construct a system in which an arrangement structure in which the direction and strength of the magnetic flux are appropriately controlled may be realized, the cell killing effect of the particle beam is maximized, the biological effect in the normal tissue 7a region through which the particle beam passes may be selectively reduced, and a damage risk is reduced.

By appropriately controlling the direction and strength of the magnetic flux, the cell killing effect of the particle beam may be maximized.

For example, when it is possible to apply a parallel magnetic field to the tumor region and apply a perpendicular magnetic field to the normal tissue 7a region, the cell killing effect on the tumor may be enhanced and the damage risk to the normal tissue 7a may be reduced at the same time.

Furthermore, the particle beam therapy may be optimized by controlling the magnetic field according to the highly radiosensitive region and the radiation resistant region in the tumor. In this manner, it is possible to obtain an effect equivalent to that in the irradiation method in which a plurality of nuclides is combined by using one nuclide, and realize an effect equivalent to that in the particle beam therapy using a plurality of nuclides.

In a case where the particle beam 3 is applied in a state in which the parallel magnetic field is applied to the spot, the secondary electrons generated around the trajectory of the particle beam 3 receives the Lorentz force by the magnetic field 9a and spirally move so as to be entangled around the trajectory and intensively emits energy in the vicinity of the trajectory. This increases the ionization density around the trajectory of the charged particles. Therefore, the cell killing effect of the particle beam 3 is enhanced than that in a case where there is no parallel magnetic field. In addition to enhancing the effect of the particle beam therapy, the cell killing effect equivalent to that of the helium beam and carbon beam may be expected, for example, by the proton beam. That is, the particle beam irradiation system using the proton beam that is inexpensive and compact in many facilities may realize the therapy equivalent to or close to the particle beam therapy that has been so far performed only by the particle beam irradiation system using the heavy particle beam that is expensive in the extremely limited number of facilities.

It has been demonstrated by the present inventors that even a small magnetic field applied to enhance the biological effect has a sufficient effect, so that the magnetic field generation device 9 for generating the magnetic field may have a simple configuration as in this example. As a result, it is possible to contribute to the miniaturization and cost reduction of the particle beam facility, implementation becomes easy, and versatility may be enhanced. However, it does not exclude introduction of magnetic field strength up to several tesla, which cannot be realized without the use of superconducting electromagnet.

By applying the magnetic field 8a, preferably the parallel magnetic field Ba to the spot to be irradiated at least while irradiating the same with the particle beam 3, the particle beam 3 may be affected and the cell killing effect may be enhanced. By applying the perpendicular magnetic field Bb on the near side of the spot to be irradiated in the irradiation direction at least while this is irradiated with the particle beam 3, the influence of the particle beam 3 on the normal cells and the like may be reduced.

The particle beam irradiation system 1 may control the biological effect of the particle beam on the irradiation target by changing the magnetic field.

The magnetic field generation device 9 may generate the magnetic field in the vicinity of the irradiation target or on the near side in the particle beam traveling direction or both of them. Therefore, it is possible to apply the magnetic field 9a in which the magnetic flux is in the direction parallel to the beam axis of the particle beam 3 to the vicinity of the target of the subject, and apply the magnetic field 9a in which the magnetic flux is in a direction perpendicular to or intersecting with the beam axis of the particle beam 3 outside the target, thereby enhancing the biological effect of the particle beam on the target and reducing the biological effect of the particle beam outside the target (on the near side of the irradiation target).

The magnetic field generation device 9 may apply the magnetic field 9a (parallel magnetic field) so as to limit the moving range of the secondary electrons to the vicinity of the trajectory of the charged particles in the vicinity of the target, so that this may enhance the biological effect of the particle beam 3.

The magnetic field generation device 9 may apply the magnetic field 9a (parallel magnetic field) so as to increase the ionization density around the trajectory of the charged particles in the vicinity of the target, thereby enhancing the biological effect of the particle beam 3.

In this manner, this particle beam irradiation system 1 is expected to make a great contribution to the industrial value and the spread of the particle beam therapy.

Note that the magnetic field may be switched in spot unit, but there is no limitation and this may be variously switched. For example, it may be configured such that, while the spots to which the same magnetic field is applied are continuous at the time of scanning irradiation with the particle beam 3, the spot irradiated with the particle beam 3 is switched without changing magnetic field setting, and the magnetic field is switched at a timing before irradiating the spot in which the magnetic field setting should be changed with the particle beam 3. In such a case, the switching of the magnetic field may be reduced.

[Example 2]

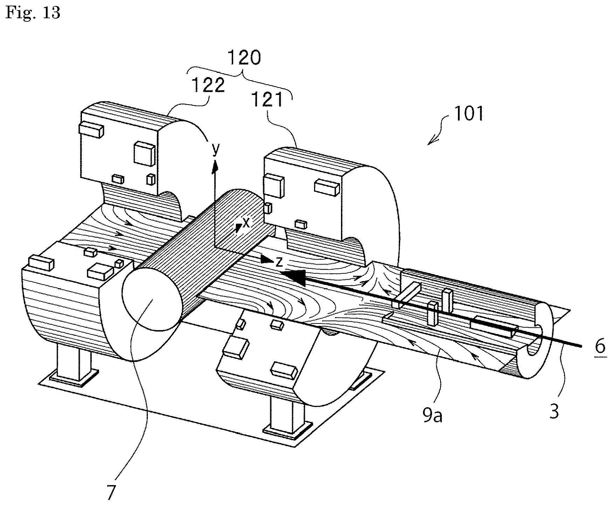

Fig. 13 is a perspective view illustrating a configuration of a magnetic field generation device 120 of a particle beam irradiation system 101 according to an example 2 of the present invention.

As illustrated in Fig. 13, the magnetic field generation device 120 includes two solenoid electromagnets (solenoid coils) 121 and 122 arranged so as to face each other on a beam axis of a particle beam. The solenoid electromagnets 121 and 122 are illustrated into a C shape partially cut in the drawing so that a magnetic line in an inner magnetic field 9a may be seen, but they are formed into the same ring shape with the same size, and are arranged separated from each other so as to be coaxial with each other. A patient 7 being an irradiation target (subject) is arranged so as to be sandwiched between the solenoid electromagnet 121 and the solenoid electromagnet 122.

The magnetic field 9a is generated by using the solenoid electromagnets 121 and 122.

The solenoid electromagnets 121 and 122 may instantly change magnetic field strength by changing a value of current applied to the solenoid coil. An irradiation direction of a particle beam 3 to a tumor of the patient 7 is not limited to a horizontal direction illustrated in the drawing, but it is also possible to rotate a rotating gantry for irradiation in a vertical direction from top to bottom. Note that it is possible to devise a shape and arrangement of the solenoid electromagnet to configure such that a parallel magnetic field or a perpendicular magnetic field is partially applied.

The strength of the magnetic field 9a applied by the solenoid electromagnets 121 and 122 is 0.6 tesla or less, but this may be increased to about 3.0 tesla as necessary. This may be performed by appropriately correcting an irradiation plan. This may also be 0.1 tesla or less in order to strengthen or weaken a required biological effect.

Other configurations and operations are the same as those of the injector 2, accelerator 4, beam transport system 5, irradiation device 6, control device 10, and irradiation planning device 20 of the particle beam irradiation system 1 of the example 1, so that the same components are assigned with the same reference signs and detailed description thereof is not repeated.

By the above-described configuration, the action and effect similar to that in the example 1 may be obtained also in the example 2.

[Example 3]

Next, an example 3 is described.

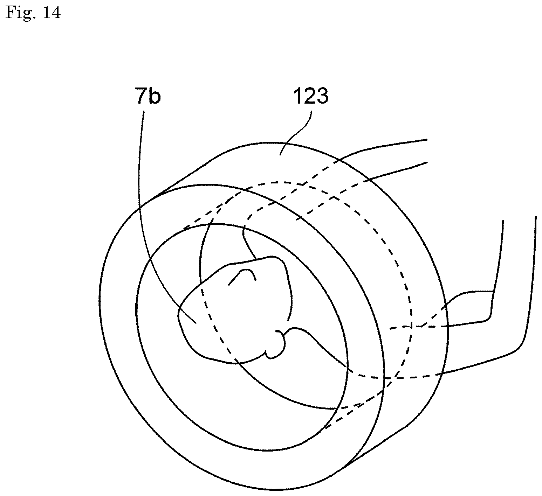

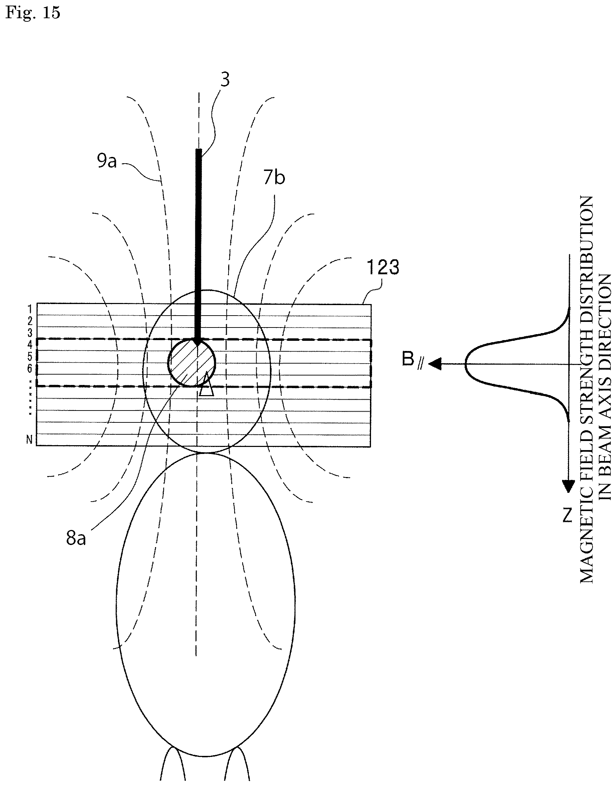

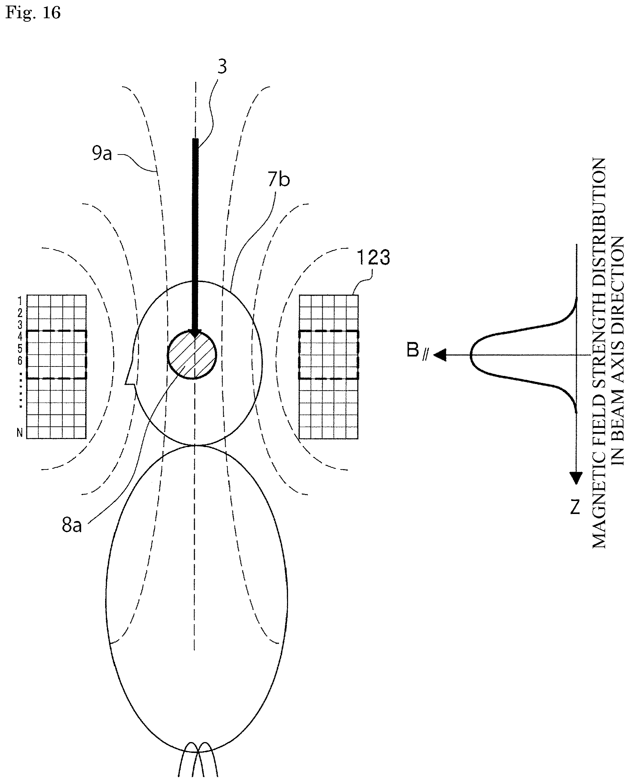

Fig. 14 is a schematic perspective view of a solenoid electromagnet 123 used in a particle beam irradiation system, Fig. 15 is a schematic configuration diagram of the solenoid electromagnet 123 in a state of surrounding head and neck 7b of a patient as seen from above, and Fig. 16 is a schematic partial cross-sectional view of the solenoid electromagnet 123 in a state of surrounding the head and neck 7b of the patient as seen from the side. Note that although a magnetic field 9a is indicated by a thin solid line in Fig. 14, the magnetic field 9a is indicated by a chain line in Figs. 15 and 16.

The solenoid electromagnet 123 (magnetic field generators 123-1, 123-2,..., and 123-N) forming a magnetic field generation device has a cylindrical shape surrounding the head and neck 7b (treated site) of the patient 7, and are arranged in a stacking manner in a beam axis direction of a particle beam 3.

It is possible to perform ON/OFF control and current value control in one layer unit of the solenoid electromagnet 123 in the stacking manner in a manner that current is applied only to the solenoid electromagnet 123 (magnetic field generators 123-4, 123-5, 123-6, and 123-7) in the vicinity of a target (in the vicinity of a tumor) indicated in broken line in Figs. 15 and 16.

As illustrated in broken line in the drawing, in a case where only the solenoid electromagnet 123 (magnetic field generators 123-4, 123-5, 123-6, and 123-7) in a layer at the same depth as the target is turned on, as illustrated in Figs. 15 and 16 as "magnetic field strength distribution in beam axis direction", a parallel magnetic field Ba is generated in parallel to the beam axis direction of the particle beam 3 by the solenoid electromagnet 123 (magnetic field generators 123-4, 123-5, 123-6, and 123-7), and a strong parallel magnetic field Ba is applied in the vicinity of a tumor 8a, whereas the magnetic field is such that the parallel magnetic field Ba is weakened and components in a perpendicular direction increase in a region of a normal tissue through which the particle beam passes.

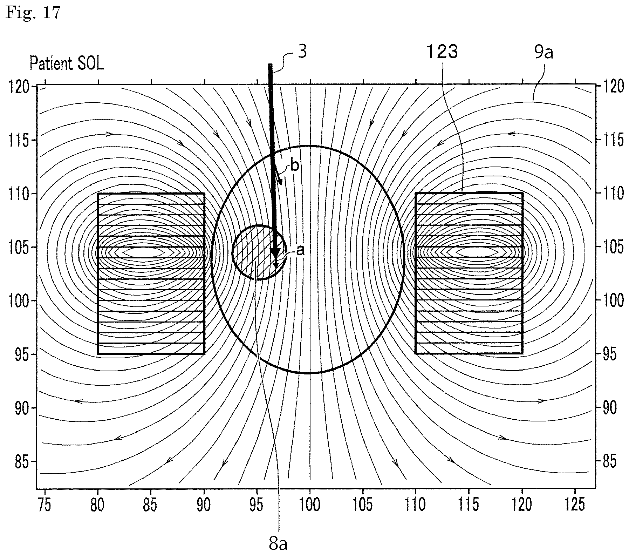

Fig. 17 is a view illustrating an example of magnetic field calculation of the solenoid electromagnet 123.

As illustrated by arrow a in Fig. 17, a "magnetic field vector in a tumor position" is parallel to the beam direction of the particle beam 3. Even in the vicinity of the tumor, a magnetic line is parallel to a beam traveling direction and the parallel magnetic field Ba is strong. As indicated by arrow b in Fig. 17, a "magnetic field vector in a normal tissue position" is oblique with respect to the beam direction of the particle beam 3. That is, in the vicinity of the normal tissue, the magnetic line is oblique with respect to the beam axis direction, a parallel component of the magnetic field is weakened, and the perpendicular component is strengthened.

In this manner, the strong parallel magnetic field Ba may be realized in the vicinity of the tumor, and the magnetic field in which the component in the perpendicular direction increases may be realized in the normal tissue region.

Other configurations and operations are the same as those of the ion source 2, accelerator 4, beam transport system 5, irradiation device 6, control device 10, and irradiation planning device 20 of the particle beam irradiation system 1 of the example 1, so that the same components are assigned with the same reference signs and detailed description thereof is not repeated.

By the above-described configuration, the action and effect similar to that in the example 1 may be obtained also in the example 3.

[Example 4]

Next, an example 4 is described.

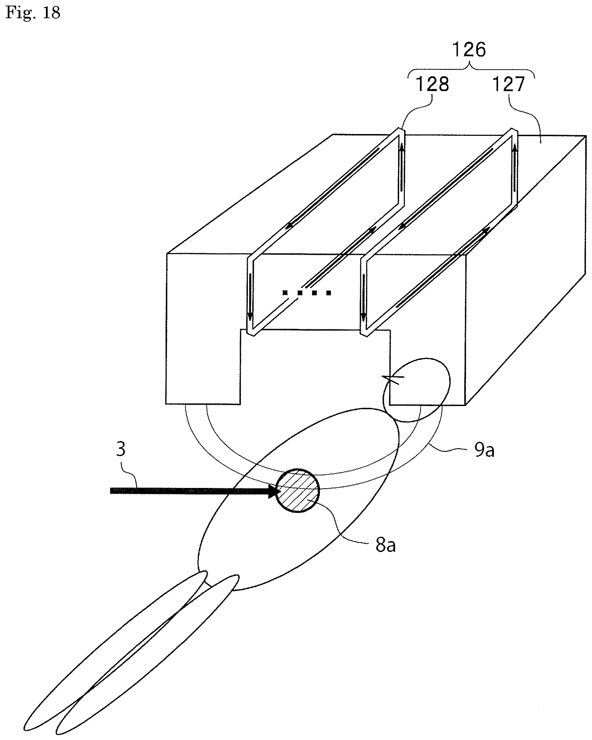

Fig. 18 is a schematic diagram illustrating a schematic configuration of an electromagnet 126 for irradiating a trunk used in a particle beam irradiation system according to the example 4.

The electromagnet 126 is formed of an inverted U-shaped iron core (return yoke) 127 with both ends of a middle part in a thick planar shape bent downward by an equal distance, and a solenoid 128 wound neatly without a gap around the middle part of the iron core 127. By applying current to the solenoid 128, the electromagnet 126 generates a magnetic field 9a.

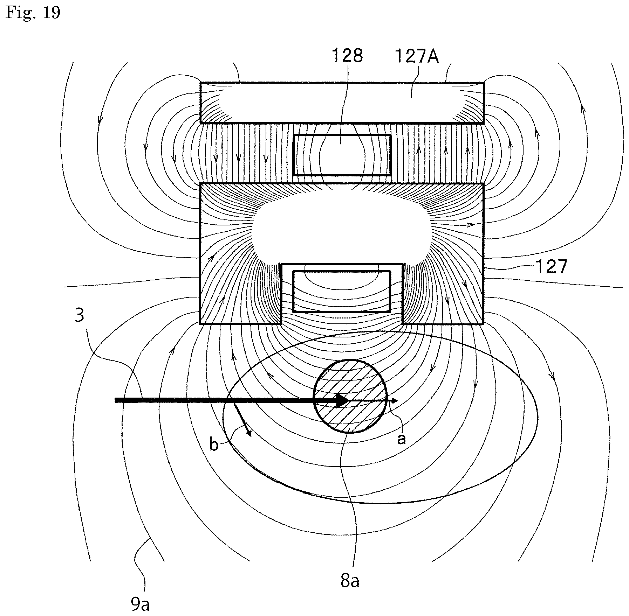

Fig. 19 is a view illustrating an example of calculating magnetic field distribution realized by the electromagnet 126 for the trunk. Above the iron core 127, that is, on a side opposite to the patient 7 of the iron core 127, an iron shield 127A having the same area as that of an upper surface of the iron core 127 is arranged in the same position as the iron core 127 in a plan view so as to be parallel to the middle part of the iron core 127.

This electromagnet 126 realizes a parallel magnetic field Ba which is the magnetic field 8a parallel to a particle beam 3 in the vicinity of a tumor 8a and a magnetic field in which components in a perpendicular direction increase in a normal tissue region. That is, in the vicinity of the tumor 8a, the parallel magnetic field Ba in which a magnetic line of the magnetic field 8a is parallel to a beam traveling direction becomes strong. In the vicinity of the normal tissue other than the tumor 8a, the magnetic line of the magnetic field 8a is oblique with respect to the beam traveling direction, so that the perpendicular component of the magnetic field strengthens.

By adjusting a positional relationship between the electromagnet 126 for the trunk and the tumor 8a of the patient according to the depth and size of the tumor, it is possible to adjust a direction and strength of a magnetic flux in the vicinity of the tumor.

Note that, although the particle beam is applied in a horizontal direction in the illustrated example, it is possible to create the parallel magnetic field Ba for the particle beam in an arbitrary direction by rotating the electromagnet 126 for the trunk according to the direction in which the particle beam is wanted to be applied.

Other configurations and operations are the same as those of the ion source 2, accelerator 4, beam transport system 5, irradiation device 6, control device 10, and irradiation planning device 20 of the particle beam irradiation system 1 of the example 1, so that the same components are assigned with the same reference signs and detailed description thereof is not repeated.

By the above-described configuration, the action and effect similar to that in the example 1 may be obtained also in the example 4.

[Example 5]

Next, an example 5 is described.

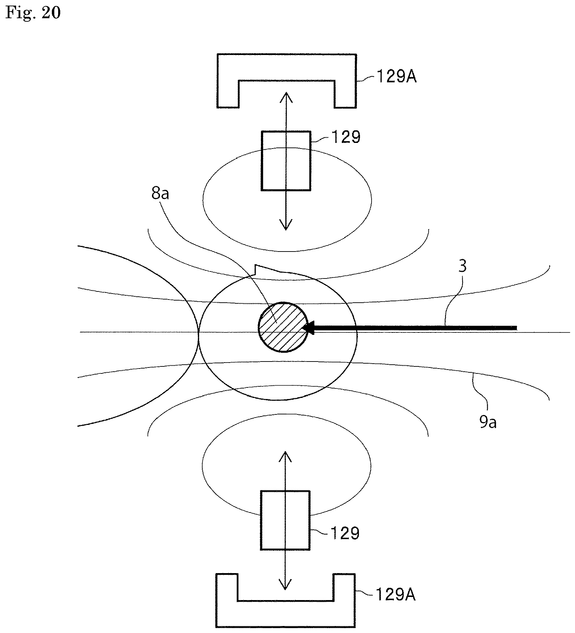

Fig. 20 is a conceptual diagram of a method of generating a parallel magnetic field using two permanent magnets 129.

The two permanent magnets 129 of the same size in a cubic shape and the same magnetic strength are arranged so as to sandwich (or surround in a case of two or more) a cancer lesion of a patient, and a distance therebetween is adjusted. That is, the two permanent magnets 129 are arranged so as to be movable in an approaching/separating direction (vertical direction in the drawing) with respect to the patient, and are configured so that a distance to the patient may be appropriately adjusted. An iron shield 129A is provided on an outer periphery of the permanent magnet 129.

In this manner, a parallel magnetic field Ba or a perpendicular magnetic field is locally applied.

Other configurations and operations are the same as those of the ion source 2, accelerator 4, beam transport system 5, irradiation device 6, control device 10, and irradiation planning device 20 of the particle beam irradiation system 1 of the example 1, so that the same components are assigned with the same reference signs and detailed description thereof is not repeated.

By the above-described configuration, the action and effect similar to that in the example 1 may be obtained also in the example 5.

[Example 6]

[Irradiation workflow: fixed port chamber]

Next, as an example 6, an example in which an irradiation workflow is made the irradiation workflow with a fixed port in the examples 1 to 5 described above is described. This case may also be used for particle beam irradiation systems 1 and 101 without a rotating gantry provided.

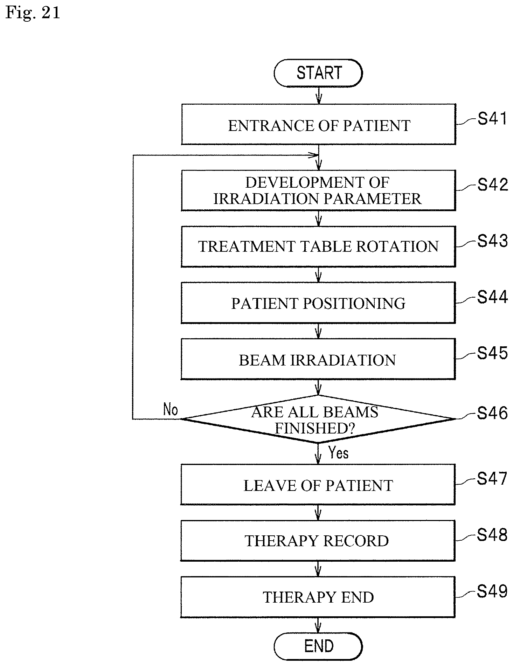

Fig. 21 is a flowchart illustrating the irradiation workflow of irradiation of a particle beam in a fixed port chamber using particle beam irradiation systems 1 and 101.

In the fixed port chamber, a therapy workflow is almost the same as that in a gantry chamber, but in the fixed port chamber, a treatment table is often rotated between first and second fields, and in this case, patient positioning is performed again after the treatment table is rotated.

A control device 10 performs the same operations as those at steps S31 to 32 described with reference to Fig. 12 in the example 1 from entry of the patient to development of an irradiation parameter (steps S41 to S42).

The control device 10 accepts the rotation of the treatment table (step S43).

The control device 10 accepts the patient positioning for beam irradiation (step S44).

From the beam irradiation to the end of therapy (steps S45 to S49), the control device 10 performs the same operations as those at steps S35 to 39 described with reference to Fig. 12 in the example 1.

Other configurations are the same as those of the ion source 2, accelerator 4, beam transport system 5, irradiation device 6, control device 10, irradiation planning device 20, and magnetic field generation devices 9 and 120 of the particle beam irradiation systems 1 and 101 of the examples 1 to 5, so that detailed description thereof is not repeated.

By the above-described configuration, the action and effect similar to that in the examples 1 to 5 may be obtained also in the example 6.

[Demonstration experiment]

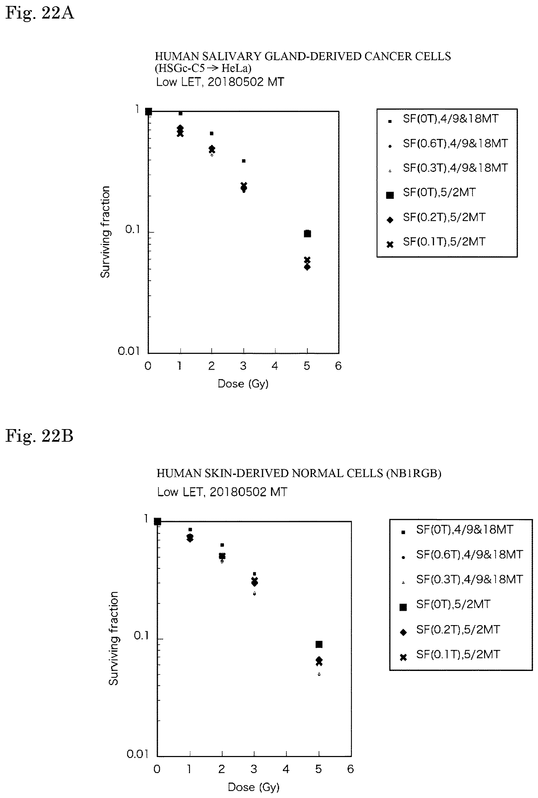

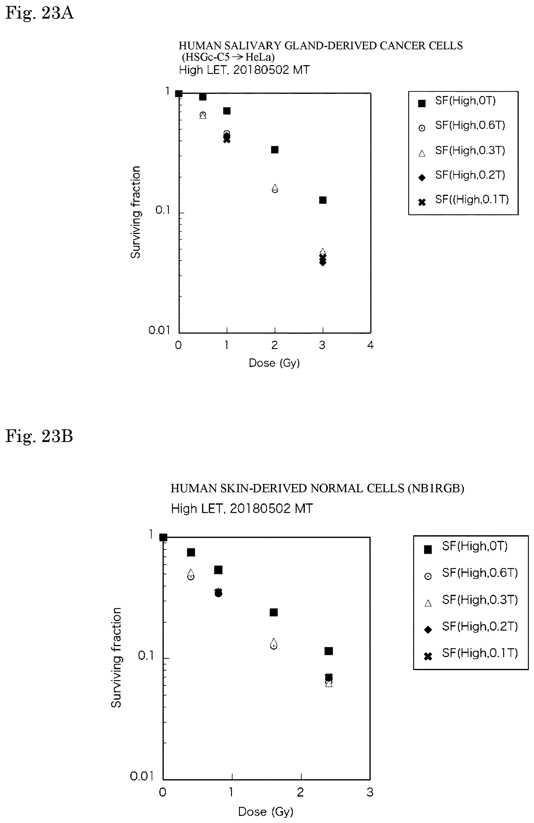

Next, a result of a demonstration experiment using one of the above-described examples is illustrated. All of Figs. 22A to 22B and Figs. 23A to 23B illustrate a dose-surviving fraction curve in which a cell surviving fraction is plotted along the ordinate and a dose is plotted along the abscissa.

Fig. 22A illustrates the dose-surviving fraction curve of cancer cells in a low LET region. The cancer cells are human salivary gland-derived cancer cells.

Fig. 22B illustrates the dose-surviving fraction curve of normal cells in the low LET region. The normal cells are human skin-derived cells.

Fig. 23A illustrates the dose-surviving fraction curve of cancer cells in a high LET region. The cancer cells are human salivary gland-derived cancer cells.

Fig. 23B illustrates the dose-surviving fraction curve of normal cells in the high LET region. The normal cells are human skin-derived cells.

As illustrated in each graph in Figs. 22A to 22B and Figs. 23A to 23B, as for the cancer cells, there was no difference in cell killing effect between a case where magnetic field strength was set to 0.1 T and 0.2 T and a case where this was set to 0.3 T and 0.6 T. That is, as for the cancer cells used in this experiment, the magnetic field of 0.1 T is sufficient to enhance the cell killing effect.

As for the normal cells, the cell killing effect in a case where the magnetic field strength was set to 0.1 T and 0.2 T was between a case where no magnetic field was applied and a case where the magnetic field of 0.3 T and 0.6 T was applied. That is, it was found that in the normal cells used in this experiment, the cell killing effect was enhanced up to about 0.3 T, and saturated above that.

In this demonstration experiment, an unexpectedly large increase in cell killing effect was observed due to a parallel magnetic field even in the vicinity of a Bragg peak of a carbon beam. This is an important finding that may change an orientation of the heavy particle beam therapy in the future.

It has been demonstrated by the experiment result that even a small magnetic field applied to enhance the biological effect has a sufficient effect, so that the magnetic field generation devices 9, 120, and 136 for generating the magnetic field may have a simplified configuration. That is, in a case of using the conventional particle beam irradiation system, the cell killing effect of the particle beam may be enhanced by adding a small magnetic field generation device, and a small and high-performance particle beam irradiation system may be obtained. In a case of constructing a particle beam facility equipped with a particle beam irradiation system capable of realizing a required cell killing effect, utilizing the small magnetic field generation device may contribute to miniaturization and cost reduction of the particle beam facility.

Since this may be realized with a magnetic field weaker than that of an MRI magnetic field generated by the MRI device, the magnetic field generation device may be downsized as compared with the MRI device, too.

Since required magnetic field strength is not so high, the present invention is easy to carry out and versatile. However, it does not exclude introduction of magnetic field strength up to several tesla, which cannot be realized without the use of superconducting electromagnet.

The above-described embodiment has been described in detail in order to explain the present invention in an easy-to-understand manner, and it is not necessarily limited to that having all the described configurations. It is possible to replace a part of the configuration of one embodiment with the configuration of another embodiment, and it is also possible to add the configuration of another embodiment to the configuration of one embodiment. It is possible to add/delete/replace another configuration as for a part of the configuration of each embodiment.

[Example 7]

Next, as an example 7, an example in which the coil shape in the example 1 is made different is described.

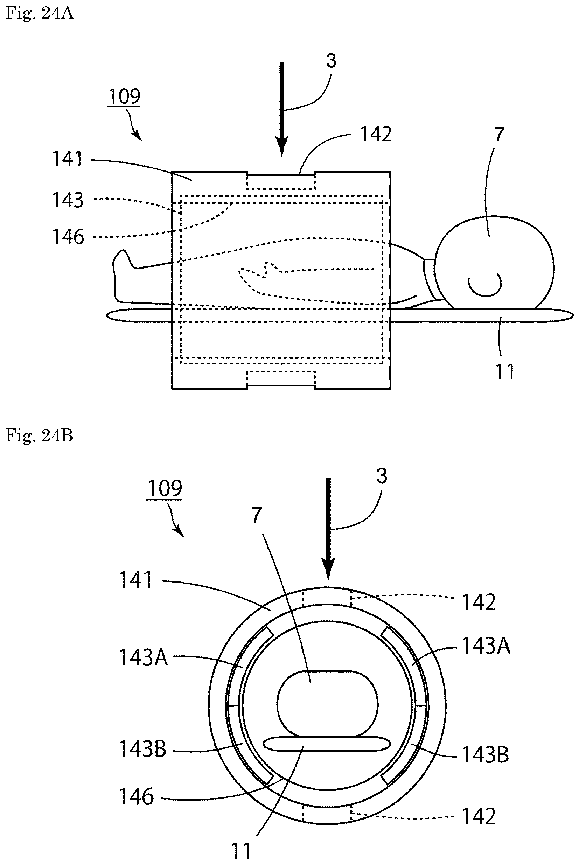

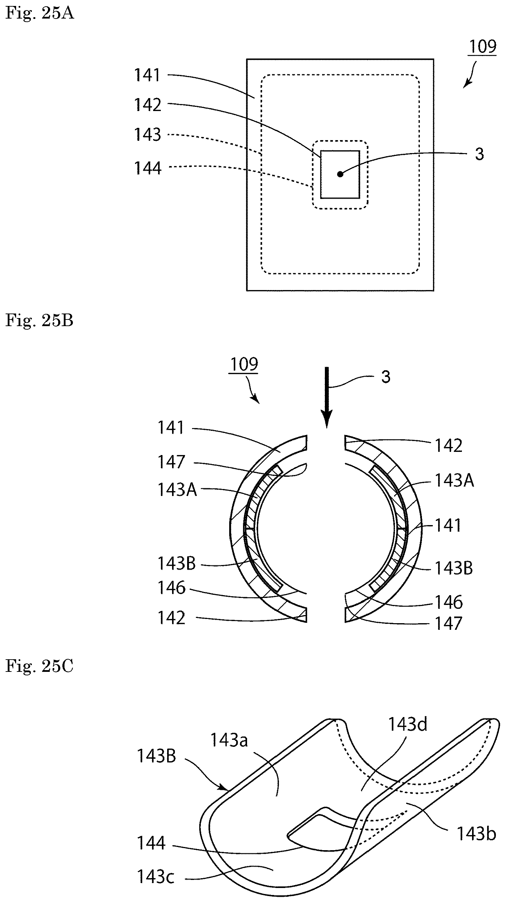

Fig. 24A is a right side view illustrating a configuration of a magnetic field generation device 109 of the example 7, Fig. 24B is a front view illustrating the configuration of the magnetic field generation device 109, Fig. 25A is a planar view illustrating the configuration of the magnetic field generation device 109, Fig. 25B is a vertical sectional view illustrating the configuration of the magnetic field generation device 109, and Fig. 25C is a perspective view illustrating a configuration of a coil 143B used in the magnetic field generation device 109.

In the magnetic field generation device 109, as illustrated in Figs. 24A and 24B, a coil 143 (143A and 143B) arranged in a cylindrical shape is arranged inside a cylindrical iron shield 141, a substantially cylindrical winding frame 146 for winding the coil 143 is arranged inside the coil 143, and a treatment table 11 in a substantially plate shape on which a person may lie is arranged inside thereof in a lower portion. The treatment table 11 is configured to be slidable inside the magnetic field generation device 109 in an axial direction of the iron shield 141 by an appropriate driving means.

The iron shield 141 is large enough for a normal person to enter the same in a lying state and particle beam through holes 142 for a particle beam 3 to pass are formed at the center on an upper surface and the center on a lower surface. The particle beam through hole 142 may have an appropriate shape such as a rectangle, a square, or a circle. For example, this may be a rectangle or a square having a side of 100 mm to 500 mm, preferably a rectangle or a square having a side of 200 mm to 400 mm, and more preferably a square having a side of 300 mm. An outer diameter size of the iron shield 141 may be set to 0.8 m to 1.6 m, preferably set to 1 m to 1.4 m, and more preferably set to 1.2 m. A length in an axial direction of the iron shield 141 may be set to 1 m to 3 m, preferably set to 1.3 m to 1.7 m, and more preferably set to 1.5 m.

The coil 143 has a size slightly smaller than that of the iron shield 141 as a whole, and is formed of semi-cylindrical upper and lower coils 143A and 143B obtained by longitudinally cutting a cylinder arranged so as to be vertically opposed to each other. As illustrated in Fig. 25C, the lower coil 143B is formed such that hollow conductors 50 (refer to Fig. 3B) are wound around a particle beam through hole 144 at the center such that a plan view and a side view are rectangular and a front view is semicircular.

The lower coil 143B includes longitudinal straight line portions 143a and 143b that are long in a trunk direction of an irradiation target (subject: patient) and arranged in parallel so as to face each other on both sides of the irradiation target to have an arc-shaped cross-section, and lateral straight line portions 143c and 143d with which both ends facing each other of the longitudinal straight line portions 143a and 143b are curved in a direction orthogonal to a longitudinal direction of the longitudinal straight line portions 143a and 143b to be connected into an arc shape in a position retracted in a direction to the arc of the arc-shaped cross-section of the longitudinal straight line portions 143a and 143b. The ends of the longitudinal straight line portions 143a and 143b are curved in an arc shape at an angle of 90 degrees along the surface of the substantially cylindrical winding frame 146 (refer to Fig. 24B), and connected to ends of the lateral straight line portions 143c and 143d curved therefrom in an arc shape along the arc shaped surface of the winding frame 146 (refer to Fig. 24B). The upper coil 143A is obtained by inverted upside down the one the same as the lower coil 143B, and is arranged so as to be opposed to the lower coil 143B.

The winding frame 146 is slightly smaller than the coil 143 and is formed in a sideways cylindrical shape, and particle beam through holes 147 through which the particle beam 3 passes are formed at the center on an upper surface and the center on a lower surface. The winding frame 146 may have an outer diameter of 600 mm to 1000 mm, preferably 700 mm to 900 mm, and more preferably 800 mm. The inside of the winding frame 146 becomes a bore, and the patient 7 may enter the same in a lying state.

Since other configurations are the same as those in the example 1, detailed description thereof is omitted.

By the above-described configuration, the action and effect the same as that in the example 1 may be obtained. Furthermore, by using the two coils 143A and 143B having the cylindrical shape so as to be opposed to each other, it is possible to generate a uniform magnetic field for the patient to be irradiated. By covering the outer periphery of the coil 143 with the iron shield 141, it is possible to efficiently generate the magnetic field and reduce a leak magnetic field to the outside.

[Example 8]

[Use of electric field]

Next, as an example 8, an example in which an electric field is used instead of a magnetic field in the examples 1 to 7 described above is described.

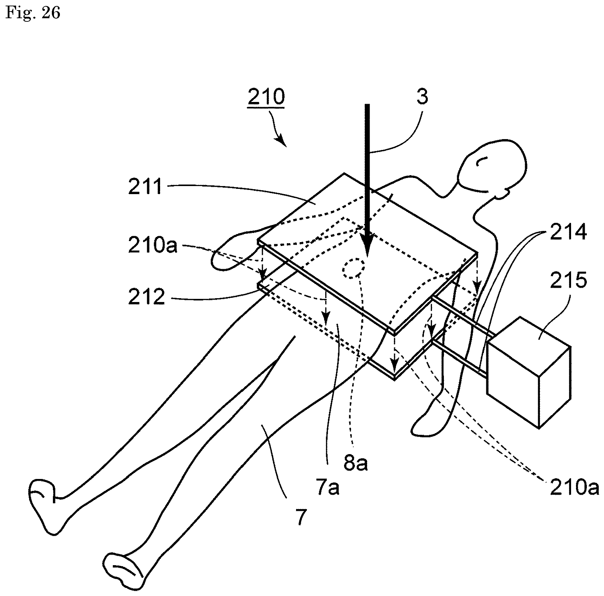

Fig. 26 is a perspective view illustrating a schematic configuration of an electric field generation device 210 of the example 8.

The electric field generation device 210 is provided with a first electrode 211, a second electrode 212, and a power control device (power supply unit) 215 that supplies electric power to the electrodes via an electric wire 214 to generate a potential difference between the electrodes to generate the electric field.

The first electrode 211 is arranged on an upper surface side (incident side of a particle beam 3) of a subject to be irradiated, and is formed into a horizontal substantially plate shape covering at least an area wider than that of a tumor 8a. It is desirable that the first electrode 211 is formed of a material that serves as an electrode such as aluminum foil, and has a size wider than a lateral width of a normal person.

The second electrode 212 is arranged on a lower surface side (emission side of the particle beam 3) of the subject to be irradiated, and is formed into a horizontal substantially plate shape covering at least an area wider than that of the tumor 8a. It is desirable that the second electrode 212 is formed of a material that serves as an electrode such as aluminum foil, and has a size wider than a lateral width of a normal person.

The first electrode 211 and the second electrode 212 have the same size and are arranged parallel to each other so that the surfaces thereof face each other.

The electric wire 214 supplies electric power from the power control device 215 to the first electrode 211 and the second electrode 212 to generate an electric field from the first electrode 211 to the second electrode 212.

The power control device 215 applies a desired electric field 210a from the first electrode 211 to the second electrode 212 according to a plan of the irradiation planning device 20 of the particle beam irradiation system 1 described in the example 1.

By this configuration, the electric field generation device 210 may uniformly generate an electric field parallel to a traveling direction of the particle beam 3 over an entire space between the first electrode 211 and the second electrode 212, thereby improving a biological effect of the particle beam.

The electric field generation device 210 configured in this manner may be used in place of the magnetic field generation device 9 in the particle beam therapeutic system 1 described in the example 1.

In this case, the control device 23 of the irradiation planning device 20 illustrated in Fig. 1 may be provided with an electric field influence operation unit instead of the magnetic field influence operation unit 38, and store electric field data in the storage device 25 instead of the magnetic field data. As the electric field data, it is preferable to store electric field distribution calculated in advance as distribution data for each material, size, separation distance, and voltage to be applied of the first electrode 211 and the second electrode 212. The electric field influence operation unit may be configured to selectively use for each material, size, separation distance, and voltage to be applied of the first electrode 211 and the second electrode 212 from the above-described distribution data. This makes it possible to calculate the influence of the electric field distribution on the particle beam 3.

It is preferable to determine arrangement of the first electrode 211 and the second electrode 212 at step S6 and calculate the electric field distribution at step S7 in the flow described with reference to Fig. 11. Then, it is preferable to calculate a pencil beam dose kernel for each electric field distribution at step S8 and determine initial values of a weight of the pencil beam and the electric field distribution at step S9. The calculation of the pencil beam dose kernel may be performed by an appropriate method such as by calculating by using electric field influence data prepared in advance by experiments and operation how much cell killing effect the particle beam of a certain dose calculation with respect to a direction and strength of the electric field.

Then, at step S10, it is preferable to calculate as an evaluation index value F, a target cell killing effect value for determining that there is a necessary and sufficient cell killing effect for the target, and an important organ cell killing effect value for determining that the cell killing effect to important organs is equal to or smaller than an allowable value in consideration of improvement/reduction of the cell killing effect by the electric field.

At step S12, the control device 23 may increment the number of repetitive calculation n (n = n + 1) in order to update the number of particles of the pencil beam and the electric field distribution (voltage value of each electrode) by the operation unit 33.

Then, at step S13, the operation unit 33 may make "the number of particles of the pencil beam with which each position is irradiated and the value of voltage applied to each electrode during the pencil beam irradiation" at that time the irradiation parameter for this field.

Since other configurations and operations are the same as those in the example 1, detailed description thereof is omitted.

In this manner, the example 8 using the electric field generation device 210 may have the action and effect similar to that of the example 1.

Note that in the particle beam therapeutic system 1 described in the example 1, it is possible to configure such that both the magnetic field generation device 9 and the electric field generation device 210 are installed, and the influence on the cell (cell killing effect and the like) by the particle beam 3 may be adjusted by using both the influence of the magnetic field of the magnetic field generation device 9 and the influence of the electric field of the electric field generation device 210. In this case, more diverse particle beam irradiation may be performed by combining both the change in the cell killing effect by the magnetic field and the change in the cell killing effect by the electric field.

The present invention is not limited to the above-described embodiment, and includes other variations and applications without departing from the gist of the present invention recited in claims.

For example, it is also possible to use an MRI magnetic field (MRI device) as a magnetic field generator. In this case, by arranging the devices such that the particle beam irradiation and imaging by the MRI do not interfere with each other, a function of image guidance by the MRI is not impaired, and the charged particle beam is not greatly deflected by the MRI magnetic field. Therefore, it is possible to maintain a function as an MRI image-guided particle beam irradiation device that irradiates the tumor with the particle beam while confirming the position thereof by the MRI.

Although the names of the particle beam therapeutic device, particle beam therapeutic system, and particle beam irradiation method are used in the above-described embodiment, this is for convenience of explanation, and the names of the devices may also be a particle beam irradiation device, a particle beam irradiation system and the like. The method may also be a particle beam therapy method and the like.

The parallel magnetic field may be a parallel component excessive magnetic field having more parallel components than the perpendicular components with respect to the irradiation direction of the particle beam, and an angle of a vector of the magnetic line with respect to the irradiation direction of the particle beam may be made smaller than 45 degrees, preferably 30 degrees or smaller, more preferably 10 degrees or smaller, and almost 0 degree is suitable. This parallel magnetic field includes a magnetic field that is completely parallel to the particle beam by a certain distance, and a curved magnetic field (substantially parallel magnetic field) having a point parallel to the particle beam 3 near the spot.

The perpendicular magnetic field may be a perpendicular component excessive magnetic field having more perpendicular components than the parallel components with respect to the irradiation direction of the particle beam, and an angle of a vector of the magnetic line with respect to the irradiation direction of the particle beam may be made larger than 45 degrees, preferably 60 degrees or larger, more preferably 80 degrees or larger, and almost 90 degree is suitable. This perpendicular magnetic field includes a magnetic field that is completely perpendicular to the particle beam by a certain distance, and a curved magnetic field (substantially perpendicular magnetic field) having a point perpendicular to the particle beam 3 near the spot or the near side of the spot (near side than the spot in the particle beam irradiation direction).

The particle beam irradiation system 1 and 101 are described as an example of a synchrotron, but there is no limitation, and this may also be a cyclotron or the like.

INDUSTRIAL APPLICABILITY

The present invention may be used in industries such as applying the particle beam.

REFERENCE SIGNS LIST

1,101: Particle beam irradiation system
6: Irradiation device
9,109,120: Magnetic field generation device
10: Control device
41,141: Iron shield
127: Iron core
43,143: Coil
121,122,123: Solenoid electromagnet (solenoid coil)
124,125,126: Electromagnet
128: Solenoid
129: Permanent magnet
150: Irradiation planning device
210: Electric field generation device
211: First electrode
212: Second electrode
215: Power control device
Ba: Parallel magnetic field
Bb: Perpendicular magnetic field

## Claims

1. A particle beam irradiation system including an irradiation device that irradiates an irradiation target with a particle beam, and an irradiation planning device that creates an irradiation plan of the particle beam by the irradiation device, the particle beam irradiation system comprising
an electromagnetic field generator that generates a magnetic field or/and an electric field that changes a cell effect that the particle beam applies to the irradiation target.

2. The particle beam irradiation system according to claim 1, wherein, based on magnetic field distribution data regarding magnetic field distribution of the magnetic field generated by the electromagnetic field generator or/and electric field distribution data regarding electric field distribution of the electric field generated by the electromagnetic field generator, the irradiation planning device is configured to calculate a cell killing effect that the particle beam applies to the irradiation target under an influence of the magnetic field distribution or/and the electric field distribution.

3. The particle beam irradiation system according to claim 2, wherein, based on the cell killing effect of the particle beam under the influence of the magnetic field distribution or/and the electric field distribution, the irradiation planning device is configured to create an irradiation plan that satisfies a condition that the cell killing effect on a target is necessary and sufficient and the cell killing effect on important organs other than the target is smaller than an allowable value.

4. The particle beam irradiation system according to claim 1, 2, or 3, wherein the electromagnetic field generator is configured to be able to generate a parallel component excessive magnetic field in which components parallel to an irradiation direction of the particle beam are more than perpendicular components at least in a spot irradiated with the particle beam.

5. The particle beam irradiation system according to claim 4, wherein
the electromagnetic field generator includes a plurality of magnetic field generators capable of being excited individually, and is configured to be able to form a plurality of types of magnetic fields while switching a magnetic field generator to be excited according to the irradiation plan of the irradiation planning device, and
the irradiation planning device is configured to determine a magnetic field generator out of the plurality of magnetic field generators to which current is applied and an amount of the current to be applied for the spot.

6. The particle beam irradiation system according to any one of claims 1 to 5, wherein the electromagnetic field generator is configured to be able to generate a magnetic field perpendicular to or intersecting with the irradiation direction of the particle beam at least in a position shallower than the spot irradiated with the particle beam.

7. The particle beam irradiation system according to any one of claims 1 to 6, wherein the electromagnetic field generator is configured to generate a magnetic field having uniform strength and magnetic flux density at least in a range irradiated with the particle beam.

8. The particle beam irradiation system according to claim 1, 2, or 3, wherein the electromagnetic field generator is formed of an electric field generation device formed of at least two electrodes arranged so as to sandwich the irradiation target and a power supply unit that applies electric power to the electrodes, and is configured to be able to generate an electric field parallel to the irradiation direction of the particle beam at least in a spot irradiated with the particle beam.

9. A particle beam irradiation method in which a particle beam irradiation system including an irradiation device that irradiates an irradiation target with a particle beam and an irradiation planning device that creates an irradiation plan of the particle beam by the irradiation device applies the particle beam, wherein an electromagnetic field generator generates a magnetic field that changes a cell effect that the particle beam applies to the irradiation target.

10. An irradiation planning program configured to allow a computer to serve as:

> a prescription data input processing unit that accepts prescription data of a particle beam;
> a magnetic field influence operation unit that calculates an influence of a magnetic field generated by an electromagnetic field generator; and
> an operation unit that calculates an irradiation parameter based on a cell killing effect of a particle beam under the influence calculated by the magnetic field influence operation unit,
> wherein the operation unit is configured to determine at least a dose and magnetic field strength of the particle beam for a region irradiated with the particle beam as the irradiation parameter.

11. An irradiation planning device that creates an irradiation plan of a particle beam in a particle beam irradiation system, comprising:

> a magnetic field data storage unit that stores magnetic field data capable of being generated by an electromagnetic field generator that generates a magnetic field; and
> an operation unit that creates a therapy plan taking into consideration of a cell killing effect of the particle beam under an influence of the magnetic field using the magnetic field data.

12. An electromagnetic field generator comprising a magnetic field generator that applies a magnetic field to a subject on a treatment table irradiated with a particle beam by a particle beam irradiation system,
the electromagnetic field generator configured to accept at least ON/OFF control of magnetic field generation by the magnetic field generator from a control device used in the particle beam irradiation system.

13. An irradiation device that irradiates an irradiation target with a particle beam based on a particle beam irradiation plan created by an irradiation planning device in a particle beam irradiation system, the irradiation device comprising:

> an electromagnetic field generator that generates a magnetic field including the irradiation target in a range; and
> a control unit that applies the particle beam in a state in which the electromagnetic field generator generates the magnetic field based on a therapy plan taking into consideration of a cell killing effect of the particle beam under an influence of the magnetic field created by the irradiation planning device.

Fig. 1

Fig. 2

Fig. 3A

Fig. 3B

Fig. 4A

Fig. 4B

Fig. 5A

7
7a
8a

CELL KILLING EFFECT

MAGNETIC FIELD

Fig. 5B

7
7a
8a

CELL KILLING EFFECT

MAGNETIC FIELD

$B_{/\!/}$  $B_{/\!/}$

Fig. 6A

Fig. 6B

Fig. 7A

7a     8a

7

CELL KILLING EFFECT

MAGNETIC FIELD

Fig. 7B

7a     8a

7

CELL KILLING EFFECT

MAGNETIC FIELD

B∥

Fig. 8A

7a    8a

7

CELL KILLING EFFECT

MAGNETIC FIELD    B⊥

Fig. 8B

7a    8a

7

CELL KILLING EFFECT

MAGNETIC FIELD    B⊥    B∥

Fig. 9

Fig. 10

Fig. 11

START

20

IRRADIATION
PLANNING DEVICE

S1 — CT IMAGING

S2 — INPUT TARGET/IMPORTANT ORGANS

S3 — DETERMINE NUMBER OF IRRADIATION FIELDS/DIRECTION

S4 — DOSE PRESCRIPTION TO TARGET/IMPORTANT ORGANS

S5 — DETERMINE PENCIL BEAM IRRADIATION POSITION

S6 — DETERMINE ELECTROMAGNET ARRANGEMENT

S7 — CALCULATION OF MAGNETIC FIELD DISTRIBUTION (PLURALITY OF TYPES ACCORDING TO VALUE OF CURRENT APPLIED TO EACH ELECTROMAGNET)

S8 — CREATE PENCIL BEAM DOSE KERNEL UNDER EACH MAGNETIC FIELD DISTRIBUTION

S9 — DETERMINE INITIAL VALUES OF WEIGHT OF PENCIL BEAM AND MAGNETIC FIELD DISTRIBUTION (CURRENT VALUE OF EACH ELECTROMAGNET)

S12 — UPDATE WEIGHT OF PENCIL BEAM AND MAGNETIC FIELD DISTRIBUTION (CURRENT VALUE OF EACH ELECTROMAGNET) $n = n + 1$

S10 — DERIVE EVALUATION INDEX VALUE F REGARDING CELL KILLING EFFECT NECESSARY AND SUFFICIENT CELL KILLING EFFECT TO TARGET, CELL KILLING EFFECT TO IMPORTANT ORGANS IS EQUAL TO OR SMALLER THAN ALLOWABLE VALUE, ENHANCEMENT/REDUCTION IN CELL KILLING EFFECT BY MAGNETIC FIELD IS TAKEN INTO CONSIDERATION IN CALCULATION OF CELL KILLING EFFECT

S11 — IS DETERMINATION $F<C$ OR $n>N$? No / Yes

S15 — UPDATE PROCESSED BEAM NUMBER $nb = nb + 1$

S13 — IRRADIATION PARAMETER (IRRADIATION POSITION OF PENCIL BEAM, NUMBER OF PARTICLES, CURRENT VALUE OF EACH ELECTROMAGNET)

S14 — IS DETERMINATION $nb = Nb$? No / Yes

S16 — THERAPEUTIC IRRADIATION

END

Fig. 12

```
              ┌─────────────┐
              │    START    │
              └──────┬──────┘
                     ▼
        ┌────────────────────────┐
        │  ENTRANCE OF PATIENT   │───S31
        └────────────┬───────────┘
                     ▼
        ┌────────────────────────┐
        │   DEVELOPMENT OF        │───S32
        │ IRRADIATION PARAMETER   │
        └────────────┬───────────┘
                     ▼
        ┌────────────────────────┐
        │  PATIENT POSITIONING   │───S33
        └────────────┬───────────┘
                     ▼
        ┌────────────────────────┐
        │   IRRADIATION PORT      │───S34
        │ (GANTRY ANGLE) SETTING  │
        └────────────┬───────────┘
                     ▼
        ┌────────────────────────┐
        │    BEAM IRRADIATION     │───S35
        └────────────┬───────────┘
                     ▼
   No        ╱ ARE ALL BEAMS ╲
  ◄─────────◄   FINISHED?      ►───S36
              ╲               ╱
                     │ Yes
                     ▼
        ┌────────────────────────┐
        │   LEAVE OF PATIENT      │───S37
        └────────────┬───────────┘
                     ▼
        ┌────────────────────────┐
        │    THERAPY RECORD       │───S38
        └────────────┬───────────┘
                     ▼
        ┌────────────────────────┐
        │    THERAPY END          │───S39
        └────────────┬───────────┘
                     ▼
              ┌─────────────┐
              │     END     │
              └─────────────┘
```

Fig. 13

Fig. 14

Fig. 15

MAGNETIC FIELD STRENGTH DISTRIBUTION
IN BEAM AXIS DIRECTION

Fig. 16

Fig. 17

Fig. 18

Fig. 19

Fig. 20

Fig. 21

```
                        ┌─────────────┐
                        │    START    │
                        └─────────────┘
                               │
                               ▼
                   ┌───────────────────────┐
                   │  ENTRANCE OF PATIENT   │──S41
                   └───────────────────────┘
                               │
          ┌────────────────────▼───────────┐
          │        ┌───────────────────────┐
          │        │   DEVELOPMENT OF       │──S42
          │        │ IRRADIATION PARAMETER  │
          │        └───────────────────────┘
          │                    │
          │        ┌───────────────────────┐
          │        │ TREATMENT TABLE ROTATION │──S43
          │        └───────────────────────┘
          │                    │
          │        ┌───────────────────────┐
          │        │  PATIENT POSITIONING   │──S44
          │        └───────────────────────┘
          │                    │
          │        ┌───────────────────────┐
          │        │    BEAM IRRADIATION    │──S45
          │        └───────────────────────┘
          │                    │
          │  No      ◇ ARE ALL BEAMS ◇──S46
          └──────────  FINISHED?
                               │ Yes
                   ┌───────────────────────┐
                   │   LEAVE OF PATIENT     │──S47
                   └───────────────────────┘
                               │
                   ┌───────────────────────┐
                   │    THERAPY RECORD      │──S48
                   └───────────────────────┘
                               │
                   ┌───────────────────────┐
                   │     THERAPY END        │──S49
                   └───────────────────────┘
                               │
                        ┌─────────────┐
                        │     END     │
                        └─────────────┘
```

Fig. 22A

HUMAN SALIVARY GLAND-DERIVED CANCER CELLS
(HSGc-C5 → HeLa)
Low LET, 20180502 MT

Fig. 22B

HUMAN SKIN-DERIVED NORMAL CELLS (NB1RGB)
Low LET, 20180502 MT

Fig. 23A

HUMAN SALIVARY GLAND-DERIVED CANCER CELLS
(HSGc-C5 → HeLa)
High LET, 20180502 MT

Fig. 23B

HUMAN SKIN-DERIVED NORMAL CELLS (NB1RGB)
High LET, 20180502 MT

Fig. 24A

Fig. 24B

Fig. 25A

141
142
143
144

109

3

Fig. 25B

109

3

141
147
143A

143B
146
142

142
143A
141
143B
146
147

Fig. 25C

143B
143a
144
143c

143d

143b

Fig. 26

Fig. 27

Fig. 28

120A

CELL
SAMPLE

110

PARTICLE BEAM

Fig. 29A

Fig. 29B

Fig. 30

MAGNETIC LINE

ELECTRON

## INTERNATIONAL SEARCH REPORT

| International application No. |
|---|
| PCT/JP2019/023963 |

### A. CLASSIFICATION OF SUBJECT MATTER
Int.Cl. A61N5/10(2006.01)i

According to International Patent Classification (IPC) or to both national classification and IPC

### B. FIELDS SEARCHED

Minimum documentation searched (classification system followed by classification symbols)
Int.Cl. A61N5/10

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>A | JP 2017-520315 A (KONINKLIJKE PHILIPS N.V.) 27 July 2017, paragraphs [0046]-[0066], all drawings & WO 2015/197475 A1, page 12, line 5 to page 16, line 4 & CN 106456994 A & EP 3160585 B1 & US 2017/0120075 A & RU 2017102494 A | 12<br>1-11, 13 |
| X<br>A | US 2001/0049475 A1 (BUCHOLZ, Richard D.) 06 December 2001, paragraphs [0040]-[0087], all drawings & EP 1121957 A2 | 12<br>1-11, 13 |
| X<br>A | WO 2017/121795 A1 (HELMHOLTZ-ZENTRUM DRESDEN-ROSSENDORF E. V.) 20 July 2017, page 29, line 14 to page 54, line 15, all drawings & DE 102016100638 A1 | 12<br>1-11, 13 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 July 2019 (05.07.2019) | 20 August 2019 (20.08.2019) |

| Name and mailing address of the ISA/<br>   Japan Patent Office<br>   3-4-3, Kasumigaseki, Chiyoda-ku,<br>   Tokyo 100-8915, Japan | Authorized officer<br><br><br>Telephone No. |

Form PCT/ISA/210 (second sheet) (January 2015)

**INTERNATIONAL SEARCH REPORT**

International application No.

PCT/JP2019/023963

| Box No. II | Observations where certain claims were found unsearchable (Continuation of item 2 of first sheet) |

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2019/023963

<Continuation of Box No. III>

(Invention 1) Claims 1-9 and 13
    Claims 1-9 and 13 have the special technical feature of an electromagnetic field generator which changes cell effects on a subject to be irradiated, and thus are classified as invention 1.
(Invention 2) Claims 10-11
    Claim 10 and claim 1 classified as invention 1 share the common technical feature of a particle beam and an apoptosis effect thereof. However, since said technical feature is a general technical feature of a particle beam treatment apparatus, and does not make a contribution over the prior art, said technical feature cannot be considered a special technical feature. In addition, there are no other identical or corresponding special technical features between these inventions. Furthermore, claim 10 is not dependent on claim 1. Further, claim 10 is not substantially identical or equivalent to any of the claims classified as invention 1. Accordingly, claim 10 cannot be classified as invention 1.
    In addition, claim 10 has the special technical feature of comprising an electromagnetic field effect calculation unit for calculating effects of electromagnetic field generated by the electromagnetic field generator in which irradiation parameters are calculated on the basis of an apoptosis effect of the particle beam under the effects calculated by the electromagnetic field effect calculation unit, and thus is classified as invention 2. Further, claim 11 has the same technical feature as that of claim 10, and thus is classified as invention 2.
(Invention 3) Claim 12
    Claim 12, and claims 1-9 and 13 classified as invention 1 and claims 10-11 classified as invention 2 share the common technical feature of irradiation of a particle beam and an electromagnetic field generator. However, said technical feature does not make a contribution over the prior art in light of documents 1-3, and thus cannot be said to be a special technical feature. In addition, there are no other identical or corresponding special technical features between these inventions. Furthermore, claim 12 is not dependent on claims 1 and 10. Further, claim 12 is not substantially identical or equivalent to any of the claims classified as invention 1 or 2. Therefore, claim 12 cannot be classified as any one of invention 1 or 2. In addition, the invention in claim 12 has the special technical feature of an electromagnetic field generator which applies an electromagnetic field to a subject on a treatment stand, and thus is classified as invention 3.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001517132 A
- JP 2008543471 A
- JP 2008543472 A
- JP 2011525390 A
- JP 2013146610 A

### Non-patent literature cited in the description

- **G.P. LINEY et al.** Technical Note: Experimental results from a prototype high-field inline MRI-linac. *Med. Phys.,* 2016, vol. 43, 5188-5194
- **B. M. OBORN et al.** Proton beam deflection in MRI fields: Implications for MRI-guided proton therapy. *Med. Phys.,* 2015, vol. 42, 2113-2124
- **T. TESSONNIER et al.** Dosimetric verification in water of a Monte Carlo treatment planning tool for proton, helium, carbon and oxygen ion beams at the Heidelberg Ion Beam Therapy Center. *Phys. Med. Biol.,* 2017, vol. 62, 6579-6594
- **T. INANIWA et al.** Treatment planning of intensity modulated composite particle therapy with dose and linear energy transfer optimization. *Phys. Med. Biol.,* 2017, vol. 62, 5180-5197